# EUROPEAN PATENT APPLICATION

(11) **EP 4 549 922 A1**
(43) Date of publication of application: **07.05.2025**
(21) Application number: 23831613.7
(22) Date of filing: 30.06.2023
(51) Int. Cl.: G01N 27/327, A61B 5/1473, A61B 5/1486, G01N 27/30, G01N 27/416

(54) **REAGENT LAYER INCLUDING CONDUCTIVE CARBON FILLER, SENSOR HAVING REAGENT LAYER, AND METHOD FOR FORMING REAGENT LAYER**

(30) Priority: 01.07.2022 JP 2022107217
(71) Applicant: PHC Holdings Corporation, Tokyo 100-8403 (JP)
(72) Inventor: HANEDA Keigo, Ehime 791-0395 (JP); HAYASHINO Nao, Ehime 791-0395 (JP)
(74) Representative: Eisenführ Speiser
(86) International application number: PCT/JP2023/024307
(87) International publication number: WO 2024/005174

(57) **Abstract**

An object of the present invention is to provide a reagent layer for preparing an electrochemical sensor that can detect an analyte with high sensitivity and/or that is suitable for measurement over a long period (continuous monitoring) and excellent in durability, and a method for forming the same. The reagent layer according to the present invention comprises a conductive carbon filler (a), an anionic dispersant (b), and a cationic mediator (c). The method for forming a reagent layer according to the present invention comprises the steps of: (1) preparing a reagent solution containing a conductive carbon filler (a), an anionic dispersant (b), and a cationic mediator (c); (2) applying the reagent solution to a reagent layer formation site; and (3) drying the applied reagent solution to form a reagent layer.

## Description

### Technical Field

The present invention relates to a reagent layer and a sensor comprising a conductive carbon filler. More specifically, the present invention relates to a conductive carbon filler dispersant as an agent for improving the dispersibility of a conductive carbon filler in an aqueous solvent, a reagent layer comprising: the dispersant, a conductive carbon filler, and a cationic mediator; a sensor having the reagent layer; and a method for forming the reagent layer.

### Background Art

Heretofore, sensors that allow proteins to act on analytes in samples and measure the analytes have been known. Examples of such a sensor include electrochemical sensors using an enzyme, for example, glucose sensors prepared using glucose oxidoreductase and optionally a redox mediator (oxidation-reduction material that mediates electron transfer) or a redox polymer (polymer having the redox mediator bound thereto via a linker or the like). The glucose sensors are used for, for example, self examination of blood glucose levels. Although conventional sensors generally involve collecting a very small amount of blood and using this blood as a sample, electrochemical glucose sensors of embedded type which are embedded in a living body to continuously measure glucose in blood or in the interstitium have also been developed in recent years. The glucose sensors are also used for measuring glucose in samples outside biological materials, for example, in media. Such glucose sensors generally perform the measurement of glucose concentrations in samples continuously or semi-continuously for a long time such as several days to several weeks.

In electrodes for use in glucose sensors typified by such electrochemical glucose sensors or biofuel batteries, conductive carbon fillers with a high specific surface area such as nanocarbon materials have been used in recent years from the viewpoint of achieving higher sensitivity or higher output by improvement in electrode specific surface area. For example, Patent Literature 1 discloses a sensor prepared from a carbon black dispersion using hydroxypropylcellulose. Patent Literature 2 discloses an enzyme-immobilized electrode obtained using ethylcellulose as a binder and carbon particles.

### Citation List

### Patent Literature

Patent Literature 1: Japanese Patent Laid-Open No. 2021-082394
Patent Literature 2: WO 2013/065581

### Summary of Invention

### Technical Problem

In general, a reagent layer comprising a conductive carbon filler and a redox mediator or a redox polymer and optionally further comprising an oxidoreductase or the like is formed on a working electrode (enzyme electrode) or the like by dispersing these necessary components in an aqueous solvent to prepare a reagent solution, and applying the reagent solution to the desired site, followed by drying. However, the conductive carbon filler generally has strong hydrophobicity and is therefore difficult to disperse in an aqueous solvent. Furthermore, a dispersed state is difficult to retain because the conductive carbon filler is susceptible to aggregation due to van der Waals' force. A reagent layer, when formed using a reagent solution containing a conductive carbon filler insufficiently dispersed therein, causes problems such as markedly reduced analyte detection sensitivity or poor durability during measurement over a long period (continuous monitoring) in a wet environment such as a living body or a medium. Dissolution of the oxidoreductase in an aqueous solvent is appropriate, and use of an organic solvent as a solvent for preparing a reagent solution is not appropriate.

In one aspect, an object of the present invention is to provide a reagent layer for preparing an electrochemical sensor that can detect an analyte with high sensitivity, and a method for forming the same. In another aspect, an object of the present invention is to provide a reagent layer for preparing an electrochemical sensor that is suitable for measurement over a long period (continuous monitoring) and excellent in durability, and a method for forming the same.

### Solution to Problem

The inventors of the present application have completed the present invention by finding that conductive carbon particles cause neither aggregation nor precipitation and have a favorable dispersed state in a reagent solution prepared using a conductive carbon filler, a cationic redox mediator or redox polymer (cationic mediator), and an anionic dispersant, preferably under specific conditions, and that since a reagent layer formed using such a reagent solution can closely adsorb the cationic mediator onto an electrode, the resulting electrochemical sensor has improved analyte detection sensitivity and is excellent in durability for continuous monitoring.

Specifically, the present invention encompasses at least the following items.

### [Item 1]

A reagent layer comprising: a conductive carbon filler (a), an anionic dispersant (b), and a cationic mediator (c).

### [Item 2]

The reagent layer according to item 1, wherein the anionic dispersant (b) is a polymer having a weight-average molecular weight of 70000 or smaller.

### [Item 3]

The reagent layer according to item 1, wherein the anionic dispersant (b) is a polymer having a carboxy group and/or a sulfo group at a side chain.

### [Item 4]

The reagent layer according to item 3, wherein the anionic dispersant (b) is a polymer comprising at least one member selected from the group consisting of an acrylic acid-derived unit, a maleic acid-derived unit, and a styrenesulfonic acid-derived unit.

### [Item 5]

The reagent layer according to item 1, wherein the cationic mediator (c) is a compound in which a redox mediator compound (c1) and a cationic polymer (c2) are bound to each other optionally via a linker moiety (c3).

### [Item 6]

The reagent layer according to item 5, wherein the cationic polymer (c2) has a quaternary ammonium cation group.

### [Item 7]

The reagent layer according to item 1, wherein the conductive carbon filler (a) is carbon black.

### [Item 8]

The reagent layer according to item 1, further comprising an oxidoreductase (e) that oxidizes or reduces an analyte.

### [Item 9]

The reagent layer according to item 8, wherein the oxidoreductase (e) is bound to a coenzyme.

### [Item 10]

The reagent layer according to item 8, wherein the oxidoreductase (e) is cross-linked to the cationic polymer (c2).

### [Item 11]

An electrochemical sensor for detecting or quantifying an analyte, the electrochemical sensor having a working electrode, a counter electrode, and the reagent layer according to any one of items 1 to 10.

### [Item 12]

The electrochemical sensor according to item 11, further having a reference electrode.

### [Item 13]

The electrochemical sensor according to item 11, further comprising a protective film with which at least the reagent layer is coated.

### [Item 14]

A method for forming a reagent layer, comprising the steps of:
(1) preparing a reagent solution containing a conductive carbon filler (a), an anionic dispersant (b), and a cationic mediator (c);
(2) applying the reagent solution to a reagent layer formation site; and
(3) drying the applied reagent solution to form a reagent layer.

### [Item 15]

The method for forming a reagent layer according to item 14, wherein a pH of the reagent solution is 8.0 or lower.

### [Item 16]

The method for forming a reagent layer according to item 15, wherein a concentration of a metal ion in the reagent solution is 200 mM or less.

### [Item 17]

The method for forming a reagent layer according to item 16, wherein the metal ion in the reagent solution is an alkali metal ion, and a concentration thereof is less than 100 mM.

### [Item 18]

The method for forming a reagent layer according to item 14, wherein the anionic dispersant (b) is a polymer having a weight-average molecular weight of 70000 or smaller and comprising at least one member selected from the group consisting of an acrylic acid-derived unit, a maleic acid-derived unit, and a styrenesulfonic acid-derived unit.

### [Item 19]

The method for forming a reagent layer according to item 14, wherein the cationic mediator (c) is a compound in which a redox mediator compound (c1) and a cationic polymer (c2) having a quaternary ammonium cation group are bound to each other optionally via a linker moiety (c3).

### Advantageous Effects of Invention

The present invention enables a conductive carbon filler to be favorably dispersed in a reagent solution containing an aqueous solvent. Use of such a reagent solution containing the conductive carbon filler dispersed therein (carbon dispersion) allows for preparation of an electrochemical sensor that achieves improvement in higher sensitivity and durability (e.g., performance that maintains response during a measurement period in measurement in a wet environment) and is excellent in workability, even though containing the conductive carbon filler.

In the present invention, whether a conductive carbon material is "dispersed" (including "redispersed") or "aggregated" (including "aggregated/precipitated") can be visually determined in most cases. For example, when a conductive carbon material is "aggregated", a floated or precipitated aggregate can be visually confirmed with ease.

Centrifugation treatment may be used for determining the dispersion and aggregation of a conductive carbon filler. For example, in the case of centrifuging a reagent solution containing a conductive carbon filler "dispersed" therein, the precipitation of the conductive carbon filler does not occur. By contrast, in the case of centrifuging a reagent solution containing an "aggregated" conductive carbon filler, an aggregate is precipitated and separated into a layer of the conductive carbon filler and a transparent aqueous solution phase. The treatment conditions of centrifugation can involve, for example, 10,000 × g and 5 minutes and may be appropriately adjusted depending on an embodiment in consideration of, for example, the type of the conductive carbon filler and its concentration.

### Brief Description of Drawings

[Figure 1] Figure 1 is a plane view of a sensor according to one embodiment of the present invention.
Figure 1(A) shows the whole sensor, and Figure 1(B) shows a tip portion of the sensor in an enlarged manner.
[Figure 2] Figure 2 is a sectional view of the sensor in a specific portion of Figure 1(B). Figure 2(A) is a sectional view taken along the AA line in Figure 1(B).
Figure 2(B) is a sectional view taken along the BB line in Figure 1(B). Figure 2(C) is a sectional view taken along the CC line in Figure 1(B).
[Figure 3] Figure 3 is a top view showing another example of the front side (side having a working electrode and a reference electrode) of the sensor according to one embodiment of the present invention.
[Figure 4] Figure 4 is a sectional view taken along the A-A' section line in Figure 3.
[Figure 5] Figure 5 is a sectional view taken along the B-B' section line in Figure 4.
[Figure 6] Figure 6 is a sectional view taken along the C-C' section line in Figure 4.
[Figure 7] Figure 7 is a plane view of a sensor according to one embodiment of the present invention.
Figure 7(A) shows an electrode pattern before film (insulating resist film) formation, and Figure 7(B) shows an electrode pattern after film formation.
[Figure 8] Figure 8 is images of recovered liquid samples that are derived from various reagent solution samples prepared in Test Example 1, and reflect a carbon black-dispersing effect. [A] Recovered liquid samples respectively corresponding to reagent solution samples 1-1 to 1-8. [B] Recovered liquid samples respectively corresponding to reagent solution samples 1-9 to 1-11.
[Figure 9] Figure 9 shows measurement results about the response of a sensor in Test Example 6. [A] Results of a current response value. [B] Results of cyclic voltammetry.
[Figure 10] Figure 10 shows measurement results about the durability of a sensor in Test Example 7. [A] Results of a sensor prepared using poly(acrylic acid). [B] Results of a sensor prepared using hydroxypropylcellulose.

### Reference Signs List

11: Sensor (probe)
21: Substrate
22: Electrode
22a: Working electrode
22b: Reference electrode
22c: Counter electrode
23: Reagent layer
24: Silver/silver chloride layer
25: Film
X1: Sensor head
X2: Direction of insertion of sensor to living body
X3: Region where counter electrode is not coated with film
X4: Region where sensor head is not coated with film
X5: Region where no reagent layer is formed
X6: Region where neither reagent layer nor electrode (working electrode) is formed (trimmed region)
101: Sensor
111: Insulating substrate
112: Conductive thin film
112a: Working electrode region
112b: Reference electrode region
112c: Counter electrode region
113: Groove
114: Working electrode
115: Reference electrode
116a: Insulating resist film (upper face)
116b: Insulating resist film (lower face)
117: Counter electrode
118: Reagent layer
119: Protective film
121: Sensing portion
122: Terminal portion
201: Sensor
202: Sensing portion
203: Terminal portion
204: Cutout portion
210: Substrate
220: Electrode
221: First working electrode
221a: Exposed region in sensing portion of first working electrode
221b: Exposed region in terminal portion of first working electrode
222: Second working electrode
222a: Exposed region in sensing portion of second working electrode
222b: Exposed region in terminal portion of second working electrode
223: Reference electrode
223a: Exposed region in sensing portion of reference electrode
223b: Exposed region in terminal portion of reference electrode
224: Counter electrode
224a: Exposed region in sensing portion of counter electrode
224b: Exposed region in terminal portion of counter electrode
225: Groove
230: Film (insulating resist film)

### Description of Embodiments

### - Reagent layer -

The reagent layer of the present invention comprises a conductive carbon filler (a), an anionic dispersant (b), and a cationic mediator (c). The reagent layer of the present invention is a layer that can be formed by a formation method using a carbon dispersion mentioned later, i.e., a reagent solution in a state where the conductive carbon filler (a) is dispersed, and is preferably a layer having a uniform state of the conductive carbon filler (a), the anionic dispersant (b), and the cationic mediator (c).

### (a) Conductive carbon filler

The "conductive carbon filler" according to the present invention is not limited to a specific type, and various conductive carbon fillers can be used in consideration of embodiments (reagent layer, application of an electrochemical sensor, etc.), working effects, etc. of the present invention. A conductive carbon material in various shapes such as a spherical (particulate), scale-like, fibrous, or porous shape can be used as the conductive carbon filler. Any one type of conductive carbon filler (a) may be used singly, or two or more types thereof may be used in combination (e.g., as a mixture).

Examples of the conductive carbon filler include carbon black, graphite powders, porous carbon materials, and nanocarbon materials. Specific examples of the carbon black include furnace black, thermal black, acetylene black, Ketjen black, and channel black. Specific examples of the graphite powder include pyrolytic graphite and spherical graphite. Specific examples of the porous carbon material include not only active carbon powders and active carbon fibers having 2 to 50 nm mesopores but carbon materials having continuous holes in which mesopores are connected. Specific examples of the nanocarbon material include single-wall carbon nanotubes, multiwall carbon nanotubes, graphene, fullerene, carbon nanohorns, and carbon nanocoils. The size of the conductive carbon filler (a) can fall within an appropriate range. For example, when the conductive carbon filler has a spherical (particulate) shape, its average particle size (e.g., an average value from a predetermined number of particles measured under an electron microscope) is generally in the range of 10 nm to 20 µm. The BET specific surface area of the conductive carbon filler is, for example, 10 m²/g or more, preferably 30 m²/g or more. The conductive carbon filler (a) according to the present invention is preferably a spherical (particulate) carbon filler, for example, carbon black.

### (b) Anionic dispersant

As used herein, the "anionic dispersant" is a compound having an essential function for dispersing the conductive carbon filler (a) in a reagent solution, and refers to a compound that is negatively charged (anionic) as a net charge of the whole molecule. Any one anionic dispersant (b) may be used singly, or two or more thereof may be used in combination (e.g., as a mixture).

However, neither "anionic surfactants" nor "anionic polysaccharides" correspond to the anionic dispersant (b) according to the present invention. Examples of the anionic surfactant include methylnaphthalenesulfonic acid formalin condensate salts (e.g., methylnaphthalenesulfonic acid formalin condensate sodium salt, product name "DEMOL MS" (Kao Corp.)), naphthalenesulfonic acid formalin condensate salts (e.g., β-naphthalenesulfonic acid formalin condensate sodium salt, product name "DEMOL N" (Kao Corp.)), alkylene-maleic acid copolymer salts (e.g., diisobutylene-maleic anhydride copolymer sodium salt, product name "DEMOL EP" (Kao Corp.)), sodium dodecyl sulfate, sodium cholate, and sodium deoxycholate. Examples of the anionic polysaccharide include anionic cellulose derivatives such as carboxymethylcellulose, anionic guar gums such as carboxymethylated guar gum, and xanthan gum. None of such anionic surfactants or anionic polysaccharides are used as the anionic dispersant (b) of the present invention.

The electrochemical sensor according to a specific embodiment of the present invention has a protective film with which the reagent layer formed on a sensor (working electrode) is coated. In such an embodiment, after formation of the protective film, the anionic dispersant (b) may be eluted and removed from the reagent layer by dipping the sensor (working electrode) in an aqueous solvent in advance, in order to prevent the anionic dispersant (b) from being leaked to the outside of the protective film (into a living body, into a medium, etc.). In such treatment, depending on the relationship between the pore size of the protective film and a molecular weight, for example, the anionic dispersant (b) bound to the conductive carbon filler (a) is not or rarely eluted (to the outside of the protective film) from the reagent layer, whereas the anionic dispersant (b) that is not bound to the conductive carbon filler (a) is eluted (to the outside of the protective film) from the reagent layer. The same as an aqueous solvent for preparing a reagent solution for use in the method for forming a reagent layer according to the present invention mentioned later can be used as the aqueous solvent in this treatment.

For example, an "anionic polymer" (excluding dispersants corresponding to anionic surfactants and anionic polysaccharides) that is a polymer having an essential function for dispersing the conductive carbon filler (a) in a reagent solution, and is negatively charged (anionic) as a net charge of the whole polymer can be used as the anionic dispersant (b) according to the present invention. The anionic polymer may have any "chain structure" of linear, branched, and comb-like forms. The "chain structure" may contain one or more ring structures derived from a cyclic compound (aromatic hydrocarbon ring, nonaromatic hydrocarbon ring, aromatic heterocyclic ring, nonaromatic heterocyclic ring, etc.). The backbone (moiety having a relatively long polymer length in the chain structure) and the side chain (moiety having a relatively short polymer length in the chain structure) of the anionic polymer are generally composed mainly of carbon atoms and may contain at least one heteroatom selected from the group consisting of a nitrogen atom, an oxygen atom, and a sulfur atom, in other words, may contain a heteroatom-containing bond such as an ether bond, a thioether bond, or an amide bond in the middle. The anionic polymer generally has a plurality of (many) negatively charged functional groups (anionic functional groups) at a side chain and may further have an anionic functional group at one or both ends of the backbone. Various polymers are known as the anionic polymer having an anionic functional group at a side chain and/or end(s). An anionic polymer having the desired properties may be purchased, or the anionic polymer may be prepared by modifying an appropriate polymer or synthesized using an appropriate monomer. The anionic polymer may be any of a homopolymer, a copolymer, and a polymer obtained by the binding and/or mixing thereof and may be any of a random polymer, a block polymer, and a graft polymer.

Examples of the anionic functional group include a carboxy group optionally having a substituent, preferably an unsubstituted carboxy group that forms -COO⁻ in an aqueous solvent, and a sulfo group optionally having a substituent, preferably an unsubstituted sulfo group that forms -SO₃⁻ in an aqueous solvent.

Examples of the anionic polymer include ethylene-based polymers having the anionic functional group at a side chain (and end(s)). Examples of the ethylene-based polymer include homopolymers and copolymers synthesized from one or two or more monomers selected from monomers containing a radically polymerizable ethylenic carbon-carbon double bond, for example, a vinyl group (CH₂=CH-), an allyl group (CH₂=CH-CH₂-), an acryloyl group (CH₂=CH-C(O) -), or a methacryloyl group (CH₂=C(CH₃) -C(O) -), and their modified forms (e.g., vinyl alcohol forms obtained by the saponification of a unit derived from vinyl acetate, and forms treated so as to impart hydrophilicity thereto). Examples of the monomer containing an ethylenic carbon-carbon double bond include ethylene, propylene, butadiene, isobutene, tetrafluoroethylene, vinyl alcohol, vinyl acetate, vinyl chloride, vinylidene chloride, styrene, methylstyrene, allylamine, diallylamine, diallyldimethylammonium chloride, acrylic acid, methacrylic acid, methyl acrylate, methyl methacrylate, butyl acrylate, butyl methacrylate, hydroxyethyl methacrylate, and acrylonitrile.

In one embodiment of the present invention, the anionic dispersant (b) is a polymer comprising at least one member selected from the group consisting of an acrylic acid-derived unit, a maleic acid-derived unit, and a styrenesulfonic acid-derived unit, i.e., a homopolymer or a copolymer synthesized using at least one monomer selected from the group consisting of acrylic acid, maleic acid, and styrenesulfonic acid (e.g., 4-styrenesulfonic acid). The copolymer may be synthesized using a monomer other than at least one monomer selected from the group consisting of acrylic acid, maleic acid, and styrenesulfonic acid (e.g., 4-styrenesulfonic acid), together with at least one monomer selected from the group consisting of acrylic acid, maleic acid, and styrenesulfonic acid (e.g., 4-styrenesulfonic acid).

In one embodiment of the present invention, the anionic dispersant (b) is a polymer having a carboxy group and/or a sulfo group as an anionic functional group at a side chain.

Preferred examples of the polymer as the anionic dispersant (b) of the present invention that can be used in both of these two embodiments include poly(acrylic acid), poly(styrenesulfonic acid), poly(styrenesulfonic acid-co-maleic acid), poly(ethylene oxide) -b-poly(acrylic acid), and poly(styrene)-b-poly(acrylic acid).

The degree of polymerization, the weight-average molecular weight, other properties, characteristics, etc. of the anionic polymer as the anionic dispersant (b) can be adjusted depending on embodiments and working effects of the present invention and the type of the anionic polymer. The degree of polymerization of the anionic polymer is usually 50 or more. The average molecular weight, typically, the weight-average molecular weight, of the anionic polymer is, for example, 1,000 or larger, preferably 5,000 or larger. The upper limit of the weight-average molecular weight of the anionic polymer is not particularly limited and is, for example, 100,000 or smaller, preferably 70,000 or smaller.

The weight-average molecular weight or the molecular weight distribution of the anionic polymer may be measured by a known approach depending on the type of the anionic polymer. For example, gel permeation chromatography (GPC) can be used. When a commercially available product is purchased and used as the anionic polymer, a numeric value shown (e.g., as "Mw" or "M.W.") in its catalog or the like can be regarded as the weight-average molecular weight.

### (c) Cationic mediator

As used herein, the "cationic mediator" is a compound (redox mediator compound) itself having an essential function as a redox mediator, or a compound comprising a site derived from such a redox mediator compound, and refers to a compound that is positively charged (cationic) as a net charge of the whole molecule. The "redox mediator" refers to an oxidation-reduction material that mediates electron transfer, and refers to, for example, a substance responsible for the transfer of electrons resulting from the redox reaction of an analyte via an oxidoreductase. Any one type of cationic mediator may be used singly, or two or more types thereof may be used in combination.

In a preferred embodiment of the present invention, the cationic mediator (c) is a compound having a structure where a "redox mediator compound" (c1) and a "cationic polymer" (c2) are bound to each other optionally via a "linker moiety" (c3). The "cationic mediator" according to the present invention, i.e., the "redox mediator", the "cationic polymer", and the "linker site" for constituting the cationic mediator, are not limited to a specific type, and various materials can be used in consideration of embodiments, working effects, etc. of the present invention.

### (c1) Redox mediator compound

The "redox mediator compound" according to the present invention is not limited to a specific type, and various redox mediators or derivatives thereof can be used in consideration of embodiments (reagent layer, application of an electrochemical sensor, etc.), working effects, etc. of the present invention.

Examples of the redox mediator compound include phenazine-based compounds, phenothiazine-based compounds, osmium complexes, ruthenium complexes, quinone compounds, and ferrocene compounds.

In one embodiment of the present invention, the redox mediator compound is a phenazine-based compound or a phenothiazine-based compound. The phenazine-based compound and the phenothiazine-based compound can be a preferred redox mediator because the compound has a negative redox potential (vs. Ag/AgCl·saturated KCl) (lower than 0 V) and is unsusceptible to impurities against electrochemical measurement contained in samples, for example, oxidizable compounds such as ascorbic acid (vitamin C) or uric acid, which are not analytes, contained in biological samples or medium samples.

The phenazine-based compound and the phenothiazine-based compound refer to compounds that have a phenazine skeleton or a phenothiazine skeleton represented by the general formulas (1) and (2), respectively, given below and are capable of functioning as redox mediators. For example, when R³ in the phenothiazine-based compound represented by the general formula (2) is a substituted amino group represented by the formula -NR³¹R³² (wherein R³¹ and R³² are both substituents, or one of these moieties is a substituent, and the other moiety is a hydrogen atom), the phenothiazine-based compound is a compound that has a resonance structure of the general formulas (2-1) and (2-2) given below and serves as a cationic mediator in itself.

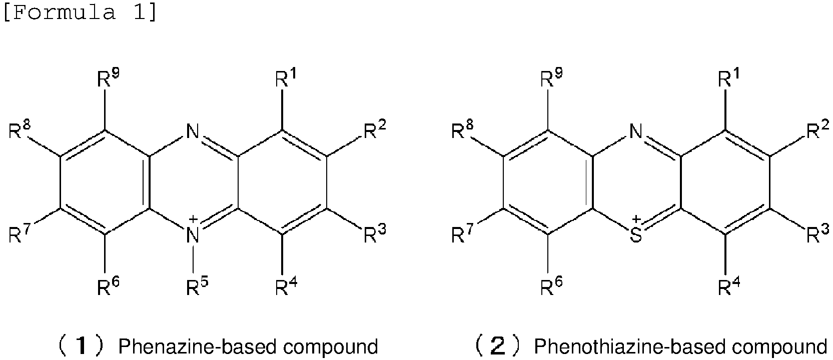

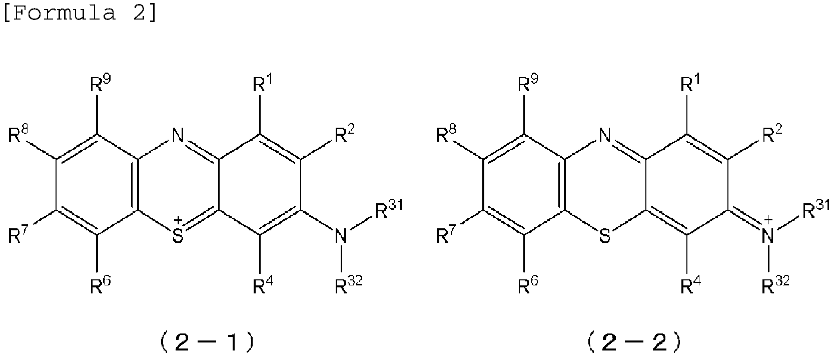

In the general formulas (1) and (2), R¹ to R⁹ each independently represent any group or atom of the following (i) to (ix') (wherein (ix') is limited to the case where the cationic mediator (c) is a compound having a structure where a "redox mediator compound" (c1) and a "cationic polymer" (c2) are bound to each other optionally via a "linker moiety" (c3), i.e., the case where (ix') reacts as a specific reactive group (which will be mentioned later in detail) of the phenazine-based compound or the phenothiazine-based compound as the redox mediator compound (c1) with a specific reactive group of the cationic polymer (c2) or a specific reactive group of a linker compound):
(i) a hydrogen atom;
(ii) a halogen atom;
(iii) a hydroxy group optionally having a substituent;
(iv) an amino group optionally having a substituent;
(v) a saturated or unsaturated hydrocarbon group optionally having a substituent (e.g., a C₁₋₁₅ alkyl group, preferably a C₁₋₆ alkyl group);
(vi) an acyl group optionally having a substituent (e.g., a C₁₋₆ acyl group, i.e., a C₁₋₆ alkyl-carbonyl group);
(vii) a phenyl group optionally having a substituent;
(viii) a quaternary ammonium cation group;
(ix) an active ester group derived from a carboxy group (e.g., an active ester group derived from a carboxy group using N-hydroxysuccinimide (NHS)); and
(ix') a carboxy group.

Examples of the substituent that may be carried by each of the substituents (iii) to (vii) include a group including at least one of (a) a halogen atom, (b) a hydroxy group, (c) an amino group, (d) a linear or branched saturated or unsaturated hydrocarbon group (e.g., a C₁₋₁₅ alkyl group, preferably a C₁₋₆ alkyl group, more preferably a C₁₋₃ alkyl group), (e) an acyl group (e.g., a C₁₋₆ acyl group, preferably a C₁₋₃ acyl group), (f) a guanidino group, (g) a mesyl group, (h) a phenyl group, (i) a thiol group, (j) a formyl group (aldehyde group), (k) an epoxy group, (l) a maleimide group, (m) an active ester group derived from a carboxy group, (m') a carboxy group, and (n) an oxyethylene group. However, the group (m') is limited to the case where the cationic mediator (c) is a compound having a structure where a "redox mediator compound" (c1) and a "cationic polymer" (c2) are bound to each other optionally via a "linker moiety" (c3), i.e., the case where (m') reacts as a specific reactive group (which will be mentioned later in detail) of the phenazine-based compound or the phenothiazine-based compound as the redox mediator compound (c1) with a specific reactive group of the cationic polymer (c2) or a specific reactive group of a linker compound. The substituent that may be carried by each of the substituents (iii) to (vii) may be a single group constituted by any of the groups (a) to (n) or may be a group constituted by two or three or more members selected from the groups (a) to (n), for example, (d) a linear or branched saturated or unsaturated hydrocarbon group further substituted by (c) an amino group, (i) a thiol group, (j) a formyl group, (k) an epoxy group, (l) a maleimide group, (m) an active ester group derived from a carboxy group, or (n) an oxyethylene group. Those skilled in the art can carry out the present invention by selecting a chemically appropriate substituent from (a) to (n) for each of the substituents (iii) to (vii) or by selecting two or three or more chemically appropriate substituents from (a) to (n) and linking these substituents. The number of substituents that may be carried by each of the substituents (iii) to (vii) is not particularly limited and may be, for example, 1, 2, or 3, or a plurality of substituents may be bonded to one atom (e.g., a carbon atom of an alkyl group or a nitrogen atom of an amino group).

The quaternary ammonium cation group (viii) refers to a group represented by -R^{A}-N⁺(R^{B}) (R^{C}) (R^{D}) (wherein R^{A}, R^{B}, R^{C}, and R^{D} each represent a saturated hydrocarbon group optionally having a substituent (e.g., a C₁₋₁₅ alkyl group, preferably a C₁₋₆ alkyl group)). The oxyethylene group (n) is the same as an oxyethylene group (e.g., a polyethylene glycol chain (PEG chain)) described in relation to the linker moiety (c3) (linker-like hydrophilic moiety).

At least one of R¹ to R⁹ may be a group for positively charging the redox mediator compound. The phenazine-based compound or the phenothiazine-based compound may have, for example, a positively charged functional group (in the present specification, referred to as a "cationic functional group") such as (iv) an amino group optionally having a substituent, preferably an unsubstituted amino group, the group forming -NH₃⁺ in an aqueous solvent, or (viii) a quaternary ammonium cation group as at least one of R¹ to R⁹ in order to render the charge of the compound alone positive (cationic).

When the cationic mediator (c) has a compound where a redox mediator compound (c1) and a cationic polymer (c2) are bound to each other optionally via a linker moiety (c3), at least one of R¹ to R⁹ may be a reactive group (in the present specification, referred to as a "specific reactive group of the redox mediator compound (c1)"; however, also simply referred to as a "specific reactive group" when the context clearly shows that the specific reactive group is carried out by the redox mediator compound (c1)) capable of binding to a group carried by the cationic polymer (c2) or the linker moiety (c3) optionally used or a linker compound for forming the linker moiety.

At least one of R¹ to R⁹ may be any of other groups related to performance as a redox mediator or groups related to the production of the reagent layer. The phenazine-based compound or the phenothiazine-based compound may have, for example, a functional group itself referred to as a so-called "hydrophilic group", such as (iii) a hydroxy group optionally having a substituent, preferably an unsubstituted hydroxy group, (iv) an amino group optionally having a substituent, preferably an unsubstituted amino group, or (viii) a quaternary ammonium cation group, a group that is derived from such a hydrophilic group and has hydrophilicity (preferably a hydrophilic group substituted by (n) an oxyethylene group or other hydrophilic groups), or a group having hydrophilicity as a whole by having (n) an oxyethylene group or other hydrophilic groups as a substituent among (v) a saturated or unsaturated hydrocarbon group optionally having a substituent, (vi) an acyl group optionally having a substituent, and (vii) a phenyl group optionally having a substituent, as at least one of R¹ to R⁹ in order to improve hydrophilicity.

As used herein, the "hydrophilicity" refers to a nature that has high affinity for water and permits dissolution or blending to the degree that a goal can be achieved in water or other polar solvents, for example, a solvent for reacting the redox mediator compound with a cationic polymer in synthesizing a cationic mediator, or a solvent for dissolving the cationic mediator in forming a predetermined layer in an electrochemical sensor. Examples of the polar solvent include methanol, ethanol, 1-propanol, 2-propanol, 1-butanol, 2-butanol, 2-methyl-1-propanol, 2-methyl-2-propanol, formic acid, acetic acid, tetrahydrofuran, acetone, dioxane, methyl ethyl ketone, ethyl acetate, acetonitrile, dimethylformamide, and dimethyl sulfoxide.

### (c2) Cationic polymer

The "cationic polymer" is a generic name for polymers having a positive charge as a net of the whole polymer. The cationic polymer (c2) according to the present invention may have a structure that can support the redox mediator compound (c1), i.e., may have a group reactable with a group carried by the redox mediator compound (c1) itself or a group carried by the linker moiety (c3) optionally used, and be capable of forming a reagent layer when applied onto the desired site, for example, onto a working electrode. The type of the cationic polymer is not particularly limited, and any one type may be used singly or two or more types may be used in combination.

The cationic polymer may have any "chain structure" of linear, branched, and comb-like forms. The "chain structure" may contain one or more ring structures derived from a cyclic compound (aromatic hydrocarbon ring, nonaromatic hydrocarbon ring, aromatic heterocyclic ring, nonaromatic heterocyclic ring, etc.). The backbone and the side chain of the cationic polymer (c2) are generally composed mainly of carbon atoms and may contain at least one heteroatom selected from the group consisting of a nitrogen atom, an oxygen atom, and a sulfur atom, in other words, may contain a heteroatom-containing bond such as an ether bond, a thioether bond, or an amide bond. The cationic polymer (c2) generally has a plurality of (many) positively charged functional groups (cationic functional groups) at a side chain and may further have a cationic functional group at one or both ends of the backbone. The cationic polymer (c2) appropriately has, at a side chain, a reactive group (in the present specification, referred to as a "specific reactive group of the cationic polymer (c2)"; however, also simply referred to as a "specific reactive group" when the context clearly shows that the specific reactive group is carried out by the cationic polymer (c2)) capable of binding to a group carried by the redox mediator compound (c1) itself or a group carried by the linker moiety (c3) optionally used or a linker compound for forming the linker moiety, and may further have a specific reactive group at one or both ends of the backbone, from the viewpoint that one molecule of the cationic polymer (c2) can support a plurality of (many) redox mediator compounds (c1) optionally via a linker moiety (c3). Various polymers are known as the cationic polymer (c2) having a cationic functional group and a specific reactive group at a side chain and/or end(s). A cationic polymer having the desired properties may be purchased, or the cationic polymer may be prepared by modifying an appropriate polymer or synthesized using an appropriate monomer. The cationic polymer (c2) may be any of a homopolymer, a copolymer, and a polymer obtained by the binding and/or mixing thereof and may be any of a random polymer, a block polymer, and a graft polymer.

Examples of the cationic polymer (c2) include ethylene-based polymers, imine-based polymers, and amino acid-based polymers having the cationic functional group and the specific reactive group at a side chain (and end(s)). The cationic polymer (c2) also encompasses a protein or a polypeptide having a natural amino acid sequence or a modified (such a modification includes substitution, deletion, addition, or the like) amino acid sequence, which can be regarded as a polymer with amino acids as monomers (on the proviso that such a protein or polypeptide is conceptually discriminated from an artificially synthesized amino acid-based polymer), and having the cationic functional group and the specific reactive group at a side chain (and end(s)). The cationic polymer (c2) also encompasses a polysaccharide-based polymer originally having the specific reactive group and the cationic functional group or having the specific reactive group and the cationic functional group introduced therein.

Examples of the ethylene-based polymer include homopolymers and copolymers synthesized from one or two or more monomers selected from monomers containing a radically polymerizable ethylenic carbon-carbon double bond, for example, a vinyl group (CH₂=CH-), an allyl group (CH₂=CH-CH₂-), an acryloyl group (CH₂=CH-C(O)-), or a methacryloyl group (CH₂=C(CH₃) -C(O) -), and their modified forms (e.g., vinyl alcohol forms obtained by the saponification of a unit derived from vinyl acetate, and forms treated so as to impart hydrophilicity thereto). Examples of the monomer containing an ethylenic carbon-carbon double bond include ethylene, propylene, butadiene, isobutene, tetrafluoroethylene, vinyl alcohol, vinyl acetate, vinyl chloride, vinylidene chloride, styrene, methylstyrene, allylamine, diallylamine, diallyldimethylammonium chloride, acrylic acid, methacrylic acid, methyl acrylate, methyl methacrylate, butyl acrylate, butyl methacrylate, hydroxyethyl methacrylate, and acrylonitrile. Preferred examples of the ethylene-based polymer also include (meth)acrylic polymers such as copolymers of methyl methacrylate and hydroxyethyl methacrylate, copolymers of butyl methacrylate and hydroxyethyl methacrylate, and poly(2-methacryloyloxyethyl phosphorylcholine-co-n-butyl methacrylate), and polyester-based polymers such as polyethylene terephthalate, which are generally known as biocompatible polymers.

Preferred examples of the ethylene-based polymer as the cationic polymer (c2) include polyallylamine hydrochloride, allylamine hydrochloride-diallylamine hydrochloride copolymers, and allylamine-diallyldimethylammonium chloride copolymers. Preferred examples of the ethylene-based polymer also include (meth)acrylic polymers having a quaternary ammonium cation, an amino group, or the like at a side chain (and end(s)). More specifically, a homo copolymer or a copolymer comprising a constituent unit derived from 2-aminoethyl methacrylate, (vinylbenzyl)trimethylammonium chloride, methacryloyl choline chloride, or the like, poly(diallyldimethylammonium chloride), poly(allylamine hydrochloride), an allylamine hydrochloride-diallylamine hydrochloride copolymer, an allylamine-diallyldimethylammonium chloride copolymer, or the like can be preferably used as the cationic polymer (c2).

Examples of the imine-based polymer include poly (ethylenimine) . The poly(ethylenimine) has a structure such as -(CH₂)₂-NH₂, -(CH₂)₂-NH-(CH₂)₂-NH₂, or - (CH₂)₂-N((CH₂)₂-NH2)₂ at a side chain, and an amino group: -NH₂ in the structure serves as the specific reactive group of the cationic polymer (c2) and can bind to the redox mediator compound (c1) or the linker moiety (c3) having an appropriate specific reactive group therefor (e.g., an active ester group derived from a carboxy group).

Examples of the amino acid-based polymer include poly(L-lysine), poly(L-arginine), and poly(L-ornithine). The poly(L-lysine) has a structure of -(CH₂)₄-NH₂ at a side chain. The poly(L-arginine) has a structure of - (CH₂)₂-NH-C(=NH)-NH₂ at a side chain. An amino group: - NH₂ (or a guanidino group: -NH-C(=NH)-NH₂) or the like contained in the side chain of such an amino acid-based polymer serves as the specific reactive group of the cationic polymer (c2) and can bind to the redox mediator compound (c1) or the linker moiety (c3) having an appropriate specific reactive group therefor (e.g., an active ester group derived from a carboxy group). The amino acid-based polymer may form a salt, for example, poly(L-arginine hydrochloride).

Examples of the polysaccharides-based polymer include cellulose derivatives such as chitosan. The chitosan is a polysaccharide obtained by hydrolyzing chitin, and has an amino group at a side chain (in a sugar structure). An amino group or the like contained in the side chain of such a polysaccharide-based polymer serves as the specific reactive group of the cationic polymer (c2) and can bind to the redox mediator compound (c1) or the linker moiety (c3) having an appropriate specific reactive group therefor (e.g., an active ester group derived from a carboxy group).

The degree of polymerization, the weight-average molecular weight, other properties, characteristics, etc. of the cationic polymer (c2) can be adjusted depending on embodiments and working effects of the present invention and the type of the cationic polymer (c2). The degree of polymerization of the cationic polymer (c2) is usually 100 or more. The average molecular weight, typically, the weight-average molecular weight, of the cationic polymer (c2) is, for example, 10,000 or larger, preferably 50,000 or larger, more preferably 100,000 or larger. The upper limit of the weight-average molecular weight of the cationic polymer (c2) is not particularly limited and is usually smaller than 10,000,000, preferably smaller than 1,000,000.

The weight-average molecular weight or the molecular weight distribution of the cationic polymer (c2) may be measured by a known approach depending on the type of the cationic polymer (c2). For example, gel permeation chromatography (GPC) can be used. When a commercially available product is purchased and used as the cationic polymer (c2), a numeric value shown (e.g., as "Mw" or "M.W.") in its catalog or the like can be regarded as the weight-average molecular weight.

### (c3) Linker moiety

The linker moiety (c3) is an optional structure for mediating the binding between the redox mediator compound (c1) and the cationic polymer (c2). The linker moiety (c3) is generally a site having a chain structure where a moiety except for reactive groups before reaction located at both ends or a bond structure after reaction is composed mainly of carbon atoms and may contain at least one heteroatom selected from the group consisting of a nitrogen atom, an oxygen atom, and a sulfur atom, in other words, a chain structure that may contain a heteroatom-containing bond such as an ether bond, a thioether bond, or an amide bond in the middle. The "chain structure" of the linker moiety (c3) may be linear or branched. The "chain structure" may contain one or more ring structures derived from a cyclic compound (aromatic hydrocarbon ring, nonaromatic hydrocarbon ring, aromatic heterocyclic ring, nonaromatic heterocyclic ring, etc.).

In one embodiment of the present invention, the linker moiety (c3) also has a function as a site (hydrophilic moiety) for improving the hydrophilicity of the cationic mediator (c) and can also be referred to as a "linker-like hydrophilic moiety". When the linker moiety (c3) is a linker-like hydrophilic moiety, the linker moiety is preferably linear and does not contain any ring structure derived from a cyclic compound, from the viewpoint of hydrophilicity.

In a preferred embodiment of the present invention, the linker-like hydrophilic moiety comprises an oxyethylene group represented by the formula: -(OC₂H₄)_{q}-. In the formula, q represents, for example, an integer of 1 to 80, preferably an integer of 3 to 36. An oxyethylene chain represented by the formula wherein q is a large number to some extent is generally called "polyethylene glycol chain (PEG chain)" or the like.

In one embodiment of the present invention, the linker-like hydrophilic moiety comprises a hydrocarbon chain represented by the formula: -(CH₂)ₚ-. In one embodiment of the present invention, the backbone of the linker-like hydrophilic moiety comprises both of a hydrocarbon chain represented by the formula: -(CH₂)ₚ- and an oxyethylene chain represented by the formula: - (OC₂H₄)_{q}-. In the formula representing a hydrocarbon chain, p can be appropriately adjusted so as to achieve a balance with q in the formula representing an oxyethylene chain in consideration of the hydrophilicity of the linker-like hydrophilic moiety.

The linker moiety (c3) can be derived from a compound (in the present specification, referred to as a "linker compound") having at least two reactive groups (in the present specification, referred to as "specific reactive groups of the linker compound"; however, also simply referred to as "specific reactive groups" when the context clearly shows that the specific reactive groups are carried out by the linker compound) capable of binding to a group (specific reactive group) carried by the redox mediator compound (c1) itself and a group (specific reactive group) carried by the cationic polymer (c2), respectively. For example, the linker compound is reacted with the redox mediator compound (c1), and then, the linker compound in a state bound to the redox mediator compound (c1) (in other words, a "redox mediator compound (c1) -linker compound reaction product") is reacted with the cationic polymer (c2) to form the cationic mediator (c) as a compound in which the redox mediator compound (c1) and the cationic polymer (c2) are bound to each other via the linker moiety (c3). Alternatively, the linker compound is reacted with the cationic polymer (c2), and then, the linker compound in a state bound to the cationic polymer (c2) (in other words, a "cationic polymer (c2) -linker compound reaction product") is reacted with the redox mediator compound (c1) to form the cationic mediator (c) as a compound in which the redox mediator compound (c1) and the cationic polymer (c2) are bound to each other via the linker moiety (c3).

### <Specific reactive group>

The specific reactive groups according to the present invention, i.e., the specific reactive group of the redox mediator compound (c1), the specific reactive group of the cationic polymer (c2), and the specific reactive groups of the linker compound can be variously combined by adopting their respective various known reactive groups. The predetermined reactive groups according to the present invention may be covalently bound to each other or may be noncovalently (e.g., through electrostatic interaction) bound to each other, and preferably, are covalently bound to each other, for example, from the viewpoint of binding stability.

The specific reactive groups according to the present invention are each independently preferably at least one member selected from the "group consisting of a carboxy group or an active ester form thereof, an amino group, a thiol group, a formyl group (aldehyde group), an epoxy group, and a maleimide group" (in the present specification, referred to as the "preferred group of specific reactive groups"). The amino group, the thiol group, the formyl group (aldehyde group), the epoxy group, and the maleimide group as preferred specific reactive groups correspond to the substituents (c), (i), (j), (k), and (l), respectively, listed as examples of the substituent that may be further carried by the substituents (iii) to (vii) as R¹ to R⁹ in the general formulas (1) and (2) regarding the redox mediator compound (c1), and the carboxy group and the active ester form thereof correspond to the substituents (m') and (m), respectively. Table 1 shows groups with which the reactive groups included in the preferred group of specific reactive groups can react respectively. In the case of selecting a reactive group included in the preferred group of specific reactive groups as one of the specific reactive groups according to the present invention, the partner group to be reacted therewith may be another reactive group included in the preferred group of specific reactive groups (underlined in the table) or may be a reactive group excluded from the preferred group of specific reactive groups (not underlined or not included in the table).

**[Table 1]**

| Reactive group | Reactive group | Formed bond, etc. |
|---|---|---|
| Carboxy group R^{a}-COOH or active ester form thereof | Amino group | Amide bond |
| | R^{b}-NH₂ | R^{a}-CO-NH-R^{b} |
| | Alcoholic or phenolic hydroxy group | Ester bond |
| | R^{b}-OH | R^{a}-CO-O-R^{b} |
| | Thiol group | Thioester bond |
| | R^{b}-SH | R^{a}-CO-S-R^{b} |
| | Alkyl halide group | Ester bond |
| | R^{b}-X | R^{a}-CO-O-R^{b} |
| Amino group R^{a}-NH₂ | Carboxy group | Amide bond |
| | R^{b}-COOH | R^{a}-NH-CO-R^{b} |
| | Acyl azide group | Amide bond |
| | Acyl halide group | Amide bond |
| | Isocyanate group | Urea bond |
| | Isothiocyanate group | Thiourea bond |
| | Imide ester | Amidine bond |
| | Epoxy group | Amide bond |
| | 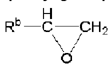 | R^{a}-NH-CO-R^{b} |
| | Alkyl halide group | Alkylamide bond |
| | Sulfonyl halide group | Sulfonamide bond |
| Thiol group R^{a}-SH | Carboxy group | Thioester bond |
| | R^{b}-COOHor active ester form thereof | R^{a}-S-CO-R^{b} |
| | Maleimide group | Thioether bond |
| | 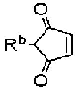 | 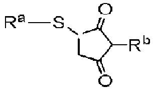 |
| | Epoxy group | Thioether bond |
| | 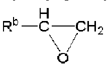 | |
| | Alkyl halide group | Thioether bond |
| | Alkyl sulfonate group | Thioether bond |
| | Aziridine group | Thioether bond |
| Formyl group (aldehyde group) R²-CHO | Alkoxyamino group | Oxime bond |
| | R^{b}-O-NH₂ | R^{a}-CH=N-O-R^{b} |
| | Amino group | Shiff base |
| | R^{b}-NH₂ | R^{a}-CH=N-R^{b} |
| | Hydrazine group | Hydrazone bond |
| | R^{b1}R^{b2}N-NH₂ | R^{a}-CH=N-NR^{b1}R^{b2} |

In a preferred embodiment of the present invention, one of the specific reactive group of the redox mediator compound (c1) or the specific reactive group of the linker compound and the specific reactive group of the cationic polymer (c2) is the "reactive group A" of Table 1, and the other group is the "reactive group B". Likewise, in a preferred embodiment of the present invention, one of the specific reactive group of the redox mediator compound (c1) and the specific reactive group of the linker compound is the "reactive group A" of Table 1, and the other group is the "reactive group B".

In a more preferred embodiment of the present invention, one of the specific reactive group of the redox mediator compound (c1) or the specific reactive group of the linker compound and the specific reactive group of the cationic polymer (c2) is an amino group, and the other group is a carboxy group or an active ester form thereof. Likewise, in a more preferred embodiment of the present invention, one of the specific reactive group of the redox mediator compound (c1) and the specific reactive group of the linker compound is an amino group, and the other group is a carboxy group or an active ester form thereof. For example, in the case of using a redox mediator compound (c1) having an active ester group derived from a carboxy group as the specific reactive group; a linker compound having an amino group as the first specific reactive group and an active ester group derived from a carboxy group (e.g., an active ester group derived from a carboxy group using NHS) as the second specific reactive group; and a cationic polymer (c2) having an amino group as the specific reactive group, first, the active ester group derived from the carboxy group of the redox mediator compound (c1) is reacted with the amino group of the linker compound (first amide bond) to obtain a redox mediator compound having a linker-like hydrophilic moiety introduced therein, and the active ester group derived from the carboxy group of the obtained redox mediator compound having a linker-like hydrophilic moiety introduced therein is reacted with the amino group of the cationic polymer (c2) (second amide bond) to obtain a cationic mediator (c).

The reagent layer of the present invention is typically disposed on a working electrode in the electrochemical sensor of the present invention. Any one type of reagent layer may be used singly, or two or more types thereof may be used in combination (e.g., two or more types of reagent layers differing in the composition (types and/or contents) of the conductive carbon filler (a), the anionic dispersant (b), and the cationic mediator (c) may be laminated).

The reagent layer may comprise an "oxidoreductase" of type appropriate for an analyte, as in an electrochemical sensor in the form of a biosensor, for example, and may comprise no oxidoreductase, as in the form of an electrochemical sensor other than the biosensor or a sensor (e.g., an optical sensor) other than the electrochemical sensor.

The reagent layer may optionally further comprise a component other than the conductive carbon filler (a), the anionic dispersant (b), and the cationic mediator (c). Examples of such an optional component include an "oxidoreductase" (d).

### (d) Oxidoreductase

The oxidoreductase (d) refers to an enzyme that can oxidize (including "dehydrogenate") or reduce an analyte targeted by an electrochemical sensor or the like. An electrochemical sensor according to the present invention typically assumes the form of an electrochemical sensor (biosensor) comprising an oxidoreductase in the reagent layer, though the electrochemical sensor is not limited thereto. The electrochemical sensor may assume the form of an electrochemical sensor other than a biosensor or a sensor (e.g., an optical sensor) other than an electrochemical sensor, comprising no oxidoreductase in the reagent layer. The presence or absence of an oxidoreductase (d) in the reagent layer can be selected depending on the application of an electrochemical sensor or the like, and the type of the oxidoreductase (d) contained can be selected depending on the type of an analyte.

Examples of the oxidoreductase (d) include oxidase-based enzymes (glucose oxidase (Gox), lactate oxidase, pyruvate oxidase, cholesterol oxidase, amino acid oxidase, glutamate oxidase, fructosyl amino acid oxidase, alcohol oxidase, ascorbate oxidase, fructosyl peptide oxidase, bilirubin oxidase, aldehyde oxidase, etc.) and dehydrogenase-based enzymes (glucose dehydrogenase (GDH), lactate dehydrogenase, pyruvate dehydrogenase, amino acid dehydrogenase, glutamate dehydrogenase, 3-hydroxybutyrate dehydrogenase, alcohol dehydrogenase, and aldehyde dehydrogenase). Any one of oxidoreductase (d) may be used singly, or two or more thereof may be used, if necessary, in combination.

In one embodiment of the present invention, the oxidoreductase (d) is bound to a coenzyme. Examples of the oxidoreductase with a reduced coenzyme (d) include GDH bound to pyrroloquinoline quinone (PQQ) and GDH bound to flavin adenine dinucleotide (FAD). In an embodiment using glucose as an analyte, GDH bound to FAD is preferred from the viewpoint that reactivity with maltose that is not an analyte is low (e.g., enzymatic activity against maltose can be 5% or less, preferably 3% or less, when enzymatic activity against glucose is defined as 100%). Examples of the GDH bound to FAD include those derived from the genus *Aspergillus* (*A. oryzae, A. terreus,* etc.) and the genus *Mucor.*

In one embodiment of the present invention, the oxidoreductase (d) may be cross-linked to the cationic mediator (c), particularly, the cationic polymer (c2) constituting it. The oxidoreductase (d) can be cross-linked to the cationic mediator (c), particularly, the cationic polymer (c2), for example, using a cross-linking agent (e). Examples of the cross-linking agent (e) include glutaraldehyde. The cross-linking agent (e) is reacted with both of a reactive group (e.g., an amino group) carried by the protein oxidoreductase (d) and a reactive group carried by the cationic mediator (c), for example, a reactive group (e.g., an amino group) carried as a reactive group that has not reacted in the cationic polymer (c2) with the redox mediator compound (c1) or the linker moiety (c2), or as a reactive group of different type from that for reaction with the redox mediator compound (c1) or the linker moiety (c2) so that the oxidoreductase (d) can be bound to the cationic mediator (c) via the cross-linking agent (e). Such cross-linking forms a composite having a larger molecular weight, and can thereby more suppress the leakage of the oxidoreductase (d) and the cationic mediator (c), particularly, the redox mediator compound (c1) constituting it to the outside of a protective film. In the case of using the cross-linking agent (e), oxidoreductases (d) may be bound to each other via the cross-linking agent (e) through reaction with reactive groups respectively carried by two molecules of the oxidoreductases (d). Specifically, in the case of using the cross-linking agent (e), in the reagent layer, the oxidoreductase (d) may be cross-linked to the cationic mediator (c), particularly, the cationic polymer (c2), while the oxidoreductases (d) may be cross-linked to each other.

### - Method for forming reagent layer -

The method for forming a reagent layer according to the present invention comprises at least the following step (1) and usually further comprises steps (2) and (3):
(1) preparing a reagent solution containing a conductive carbon filler (a), an anionic dispersant (b), and a cationic mediator (c) (in the present specification, also referred to as a "reagent solution preparation step");
(2) applying the reagent solution to a reagent layer formation site (in the present specification, also referred to as an "application step"); and
(3) drying the applied reagent solution to form a reagent layer (in the present specification, also referred to as a "reagent layer formation step").

### (1) Reagent solution preparation step

The reagent solution preparation step (1) is the step of preparing a reagent solution containing main components constituting the reagent layer, i.e., at least a conductive carbon filler (a), an anionic dispersant (b), and a cationic mediator (c), and optionally containing other components, for example, an oxidoreductase (d) and a cross-linking agent (e).

The reagent solution can generally be prepared by adding necessary components among the components (a) to (e) to an aqueous solvent (water or a mixed solvent of water and a solvent compatible with water). The aqueous solvent can be adjusted so as to have an appropriate pH, if necessary, by the addition of a pH adjuster, a buffer (solution), other compounds, or the like.

In one embodiment of the present invention, the pH of the reagent solution is 8.0 or lower. Use of a buffer solution in an amount that adjusts the pH of the reagent solution to 8.0 or lower is more effective for improving the dispersibility of the conductive carbon filler (a). When the reagent solution contains an oxidoreductase (d), the pH of the reagent solution is appropriately in a range that maintains the activity of the oxidoreductase (d).

The pH of the reagent solution is usually adjusted using a buffer solution. Various buffer solutions can be used. Examples of the buffer solution include acetate buffer solutions (acetic acid and sodium acetate), phosphate buffer solutions, citrate buffer solutions, citrate-phosphate buffer solutions, Tris buffer solutions (trishydroxymethylaminomethane, also called trometamol), Bis-Tris buffer solutions (bis(2-hydroxyethyl)iminotris(hydroxymethyl)methane), HEPES buffer solutions (2-[4-(2-hydroxyethyl)-1-piperazinyl]ethanesulfonic acid), MES (2-morpholinoethanesulfonic acid monohydrate), MOPS (3-morpholinopropanesulfonic acid), and PBS (phosphate-buffered saline; generally sodium chloride, potassium chloride, disodium hydrogen phosphate, and potassium dihydrogen phosphate). Use of an appropriate buffer solution (buffer) can adjust the pH of the reagent to the desired value (range).

In one embodiment of the present invention, the concentration of a metal ion in the reagent solution is 200 mM or less. When the reagent solution has a high concentration of a metal ion, the metal ion, depending on its type, might inhibit the dispersibility of the conductive carbon filler and cause aggregation. Therefore, a lower concentration of the metal ion in the reagent solution tends to be more preferred. In the case of using a compound (buffer) containing a metal ion or a buffer solution prepared therefrom for the pH adjustment of the reagent solution, the concentration of the metal ion in the reagent solution is calculated on the basis of the amount of the buffer or the buffer solution necessary for attaining the desired pH, and the concentration is preferably set to 200 mM or less, or in other words, a buffer or a buffer solution that can attain 200 mM or less is preferably selected. In the case of using a compound (buffer) containing no metal ion or a buffer solution prepared therefrom, the concentration of a metal ion in the reagent solution can result in 200 mM or less. When a metal salt is an alkali metal ion, the concentration of the metal ion in the reagent solution is preferably less than 100 mM, for example, 80 mM or less.

Since the metal ion in the reagent solution may be derived from various substances for use in the preparation of the reagent solution, the composition of the reagent solution, the type of each component or the like, and the amount of each component or the like used can be adjusted such that the concentration of the metal ion falls within an appropriate range. For example, since the metal ion in the reagent solution may be derived from a buffer solution, the buffer solution for use in the pH adjustment of the reagent solution can have appropriate composition (type and concentration of a compound contained in the buffer solution). Also since the metal ion in the reagent solution may be derived from a salt formed as the anionic dispersant (b), the amount of the anionic dispersant (b) forming such a salt can be taken into consideration, if necessary.

The order of addition of the components to be contained in the reagent solution can be appropriately adjusted and is preferably, for example, the following order of the steps (1-1) and (1-2):
(1-1) dispersing the conductive carbon filler (a) in an aqueous solution containing at least the anionic dispersant (b) to obtain a dispersion; and
(1-2) adding the cationic mediator (c) to the obtained dispersion to obtain a reagent solution.

The respective concentrations of the conductive carbon filler (a), the anionic dispersant (b), and the cationic mediator (c), and the oxidoreductase (d) optionally used in the reagent solution, or the ratio between the mutual concentrations can be appropriately adjusted in consideration of embodiments, working effects, etc. of the present invention.

The concentration of the conductive carbon filler (a) in the reagent solution is in the range of, for example, 0.1 to 30 w/v% (mg/mL).

The concentration of the anionic dispersant (b) in the reagent solution is in the range of, for example, 0.01 to 30 w/v% (mg/mL).

In the reagent solution preparation step (1), treatment for improving the dispersibility of the conductive carbon filler (a) in the reagent solution (in the present specification, also referred to as "dispersion treatment") may be performed by applying physical energy to the reagent solution. The dispersion treatment can be performed, for example, between the step (1-1) and the step (1-2) and may be performed after the step (1-2) (before the subsequent application step (2)) instead thereof or in addition thereto.

The approach (method and conditions therefor, apparatus used, etc.) for applying the "physical energy" is not particularly limited as long as the dispersibility of the conductive carbon filler (a) in the reagent solution can be improved. In a typical embodiment of the present invention, ultrasonication is used as the approach described above, though other approaches may be used. The conditions (output, time, etc.) for ultrasonication can be appropriately adjusted. The apparatus for ultrasonication can also be appropriately selected. For example, an ultrasonic homogenizer can be used.

The oxidoreductase (d) is preferably added at a stage where dispersion treatment is finished in the reagent solution preparation step (1) such that the oxidoreductase is not influenced by the physical energy. The cross-linking agent (e), for example, glutaraldehyde, for cross-linking the oxidoreductase (d) to the cationic mediator (c) or cross-linking the oxidoreductases (d) to each other is preferably added after addition of the oxidoreductase (d).

In the case of using, as the cationic mediator (c), a compound in which a redox mediator compound (c1) and a cationic polymer (c2) are bound to each other optionally via a linker moiety (c3), the cationic mediator (c) is preferably added after the dispersibility of the conductive carbon filler (a) is improved by applying physical energy to the reagent solution by dispersion treatment, for example, ultrasonication.

### (2) Application step

The application step (2) is the step of applying (adding dropwise, etc.) the reagent solution obtained by the reagent solution preparation step (1) to a reagent layer formation site, for example, at least a portion of a working electrode.

The approach (method and conditions therefor, apparatus used, etc.) for "application" is not particularly limited as long as the reagent layer can be formed from the reagent solution. An approach that conforms to a reagent solution application step in a conventional method for forming a reagent layer (or method for producing an electrochemical sensor) can be adopted. In a typical embodiment of the present invention, the application step is performed by adding dropwise an appropriate amount of the reagent solution obtained by the reagent solution preparation step (1) to at least a portion (portion where the reagent layer is to be formed) of a working electrode. The area for application and the amount of the reagent solution applied can be adjusted depending on the thickness of the reagent layer of interest.

### (3) Reagent layer formation step

The reagent layer formation step (4) is the step of drying the reagent solution applied to the reagent layer formation site (e.g., at least a portion of a working electrode) by the application step (2) to form a coated layer.

The approach (method and conditions therefor, apparatus used, etc.) for "drying" is not particularly limited as long as the (preferably, uniform) reagent layer can be formed from the applied reagent solution. An approach that conforms to a reagent solution drying step in conventional production of an electrochemical sensor can be adopted. In a typical embodiment of the present invention, the reagent layer is formed through the reagent layer formation step performed by leaving the reagent solution-applied working electrode standing at room temperature.

### - Electrochemical sensor -

The electrochemical sensor for detecting or quantifying an analyte according to the present invention has a working electrode, a counter electrode, and the reagent layer of the present invention as mentioned above disposed on the working electrode, and optionally further has a protective film with which at least the reagent layer is coated.

The "protective film" is a membrane-like member for preventing or suppressing the leakage of a substance (the conductive carbon filler (a), the cationic mediator (c), the oxidoreductase (d) optionally used, etc.) contained in the reagent layer into an environment (into a living body, into a medium, etc.) outside the protective film. The protective film with which the reagent layer is coated appropriately has pores through which an analyte present in an environment outside the protective film is capable of penetrating the protective film so as to come into contact with the reagent layer. Such a protective film is preferably formed, for example, when the electrochemical sensor is used in sustained measurement, and may not be formed when the electrochemical sensor is used in single measurement.

The working electrode (or a probe having this electrode) on which the reagent layer is formed is inserted to a living body for use or dipped in a medium for use. It is therefore preferred that the protective film which coats the surface thereof have biocompatibility by which proteins or cells are not adsorbed thereto or are difficult to adsorb thereto, and generally be formed from a biocompatible polymer having such properties. Examples of the biocompatible polymer include copolymers of methyl methacrylate and hydroxyethyl methacrylate, copolymers of butyl methacrylate and hydroxyethyl methacrylate, and poly(2-methacryloyloxyethylphosphoryl choline-co-n-butyl methacrylate).

The protective film can be formed by preparing a solution containing a starting material for forming the protective film (protective film solution), dipping a region where the protective film is to be formed, for example, a region provided with at least the reagent layer on the working electrode, in the solution, pulling up and drying the resultant, and, if necessary, repeating such a process a plurality of times.

The electrochemical sensor of the present invention may optionally further comprise a reference electrode. Specifically, the electrochemical sensor of the present invention may be prepared into a two-electrode system constituted by a working electrode and a counter electrode or may be prepared into a three-electrode system constituted by a working electrode, a counter electrode, and a reference electrode.

In one embodiment of the present invention, the electrochemical sensor can be prepared as an electrochemical sensor of embedded type, for example, a biosensor for CGM (continuous glucose monitoring) for self monitoring of blood glucose which continuously or semi-continuously measures glucose concentrations in blood or interstitial liquid, for example, over several days to several weeks. On the other hand, in one embodiment of the present invention, the electrochemical sensor can also be prepared as an electrochemical sensor of nonembedded type, for example, a biosensor for continuously or semi-continuously measuring concentrations of glucose or the like in a medium.

Hereinafter, embodiments of the electrochemical sensor of the present invention of embedded type will be described with reference to the drawings. However, the drawings and the following description are given for illustrative purposes and do not limit the technical scope of the present invention to the embodiments disclosed thereby. It should be understood that variously changed or modified embodiments are included in the scope of the present invention as long as the objects of the present invention can be attained and as long as the working effects of the present invention are exerted.

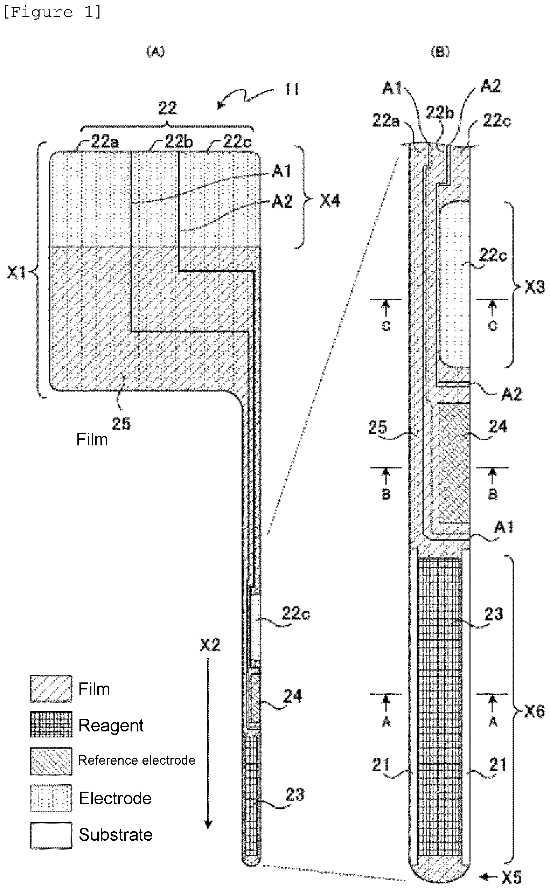

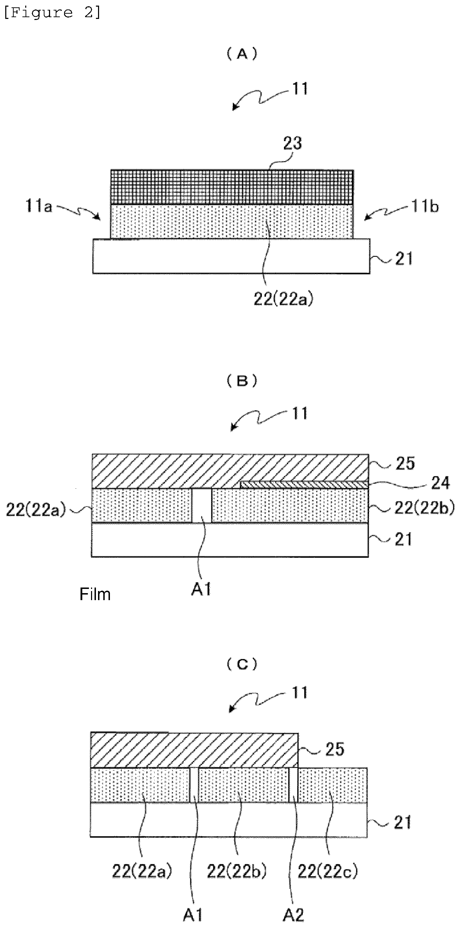

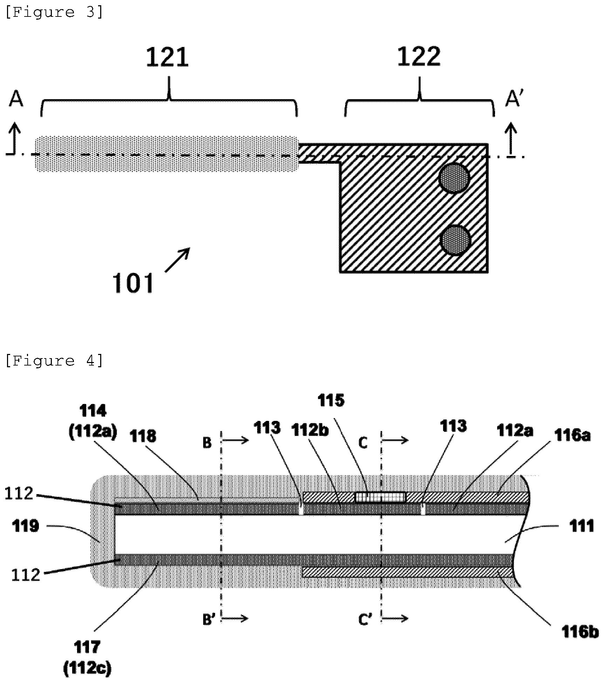

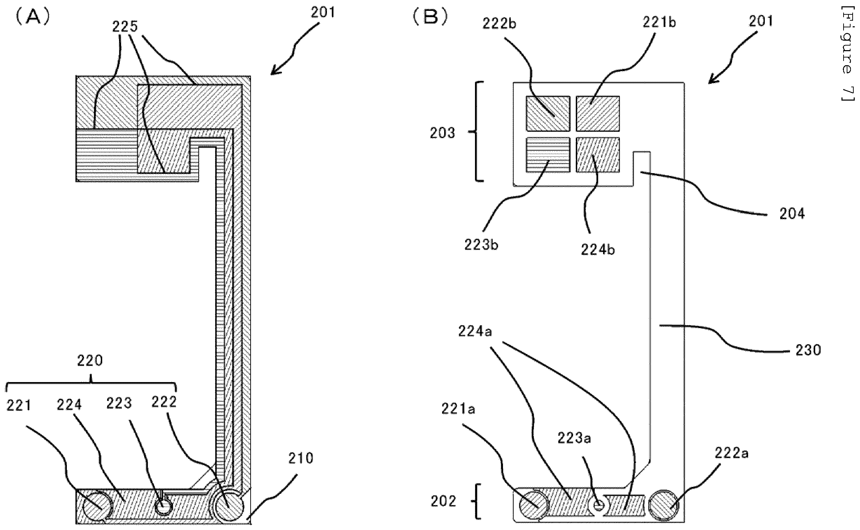

In the description below, a face directed out of the paper in Figures 1(A), 1(B), 3, and 7 or a face directed upward on the paper in Figures 2(A) to 2(C) and 4 to 6 is also referred to as an "upper face"; a face directed into the paper in Figures 1(A) and 1(B), 3, and 7 or a face directed downward on the paper in Figures 2(A) to 2(C) and 4 to 6 is also referred to as a "lower face"; and a face directed to either the left or the right in Figures 1(A), 1(B), 2(A) to 2(C), and 5 to 7 or a face directed either out of or into the paper in Figure 4 is also referred to as a "side face". A dimension in the upward and downward direction of the paper in Figure 1(A), i.e., the direction parallel to the arrow X2 which indicates the direction of insertion of the sensor to a living body, and a dimension in the upward and downward direction of the paper in Figures 3 and 7 are each also referred to as a "length", and a dimension in the left and right direction of the paper in Figure 1(A), i.e., the direction perpendicular to the arrow X2, and a dimension in the upward and downward direction of the paper in Figures 3 and 7 are each also referred to as a "width".

Figure 1 is a plane view of a sensor 11 according to one embodiment of the present invention. Figure 1(A) shows the whole sensor, and Figure 1(B) shows a tip portion (sensing portion) of the sensor 11 shown in Figure 1(A) in an enlarged manner. The sensor 11 is suitable for constructing, for example, a system of a biosensor of embedded type which is attached to a living body for use in self examination of blood glucose levels. In this case, the sensor 11 can be inserted as a protrusion (probe) from the main body (not shown) to a living body. The sensor 11 can also be used for constructing, for example, a system for measuring the concentration of an analyte in a medium.

The region X1 (head) of the sensor 11 shown in Figure 1(A) is housed in the main body (not shown), and the tip portion (sensing portion) of the sensor 11 protrudes from the main body. Arrow X2 indicates the direction of insertion, for example, when the sensor 11 is inserted to a living body. The dimension of the sensing portion is a length of, for example, 20 to 3 mm, preferably 10 to 3 mm, and a width of, for example, 1 to 50 ·µm, preferably 500 to 50 µm.

In Figure 1, the sensor 11 has a substrate 21, an electrode 22, a reagent layer 23, a silver/silver chloride layer (also referred to as reference layer) 24, and a film 25. The electrode 22 is uniformly formed on the substrate 21 and comprises a working electrode 22a, a reference electrode 22b, and a counter electrode 22c. The working electrode 22a and the reference electrode 22b are separated physically and electrically through a groove A1, and the reference electrode 22b and the counter electrode 22c are separated physically and electrically through a groove A2. The reagent layer 23 is formed on the working electrode 22a. The silver/silver chloride layer 24 is formed on the reference electrode 22b. The upper face of the sensor 11 except for a portion (in the region X1 of the head, a region X4, a region X3 of the counter electrode 22c, etc.) of the electrode 22 and the reagent layer 23 (by having an opening that exposes them) is coated with the film 25. The exposed region X4 of the electrode 22 is connected with a circuit of the main body 11.

As indicated by a region X5 in Figure 1(B), it is preferred that no reagent layer 23 is formed (over a predetermined distance from the tip) at the tip of the sensor 11. In other words, it is preferred that the reagent layer 23 be distantly formed from the tip of the sensor 11. This is because the detachment (peeling) of the reagent layer 23 from the sensor 11 can thus be suppressed when the sensor 11 is inserted to a living body.

Figure 2(A) is a sectional view taken along the AA line in Figure 1(B). In a portion where the reagent layer 23 is formed in the sensor 11, the substrate 21, the electrode 22 (the working electrode 22a), and the reagent layer 23 are laminated in this order. In portions 11a and 11b, the electrode 22 (the working electrode 22a), the reagent layer 23, and the film 25 are not laminated (are trimmed) to expose the substrate 21.

Figure 2(B) is a sectional view taken along the BB line in Figure 1(B). In a portion where the silver/silver chloride layer 24 is formed on the right side with respect to the groove A1, the substrate 21, the electrode 22 (the reference electrode 22b), the silver/silver chloride layer 24, and the film 25 are laminated. A portion separated physically and electrically from the reference electrode 22b on the left side with respect to the groove A1, the substrate 21, the electrode 22 (the working electrode 22a), and the film 25 are laminated. The side face (right side face in Figure 2(B)) of the silver/silver chloride layer 24 is exposed without being provided with the film 25. In the present embodiment, the upper face of the silver/silver chloride layer 24 is coated with the film 25 or may be exposed without being coated with the film 25.

Figure 2(C) is a sectional view taken along the CC line in Figure 1(B). In a portion on the right side with respect to the groove A2, the substrate 21 and the electrode 22 (the counter electrode 22c) are laminated. The upper face of the counter electrode 22c is exposed without being coated with the film 25. In a portion flanked by the groove A1 and the groove A2, the substrate 21, the electrode 22 (the reference electrode 22b), and the film 25 are laminated. In a portion on the left side of the groove A1, the substrate 21, the electrode 22 (working electrode 22a), and the film 25 are laminated.

The substrate 21 is typically a sheet-shaped synthetic resin. The material of the substrate 21 is not particularly limited as long as the material is a resin material such as a plastic material (synthetic resin) having at least one or more features of flexibility, processability, and heat resistance. Typical examples of such a resin material of the substrate 21 include polyethylene terephthalate (PET). Other examples thereof include general-purpose plastics such as polyethylene, polypropylene, and polyethylene naphthalate. Polyimide is also preferred If high heat resistance is required.

The electrode 22 is a thin film (thin layer) formed on the substrate 21. The material of the electrode 22 is not particularly limited as long as the material is a metal or carbon material having conductivity and stability (e.g., oxidation resistance or salt tolerance). Typical examples of such a material of the electrode 22 include gold. Other examples thereof include platinum, palladium, and carbon. In the case of forming the working electrode 22a (the reagent layer 23) on one of the upper face and the lower face of the sensor 11 and forming the counter electrode 22c on the other face, electrode materials different from each other may be used.

The working electrode 22a is given a potential (potential based on the reference electrode 22b) sufficient for oxidizing a mediator reduced through the reaction of an analyte (e.g., glucose) with an oxidoreductase. Glucose concentrations are measured by monitoring a current flowing between the working electrode 22a and the counter electrode 22c. The reagent layer 23 is formed on the upper face of the working electrode 22a in the tip portion of the sensor 11.

The silver/silver chloride layer 24 is formed, if necessary, on the upper face of the reference electrode 22b in the tip portion of the sensor 11. In the present embodiment, an exemplary three-electrode configuration consisting of the working electrode 22a, the reference electrode 22b, and the counter electrode 23c is shown so as to achieve more accurate measurement. A two-electrode configuration excluding the reference electrode 22b and consisting of the working electrode 22a and the counter electrode 23c is also possible (e.g., such a two-electrode configuration is mainstream for currently commercially available SMBG (machine for self monitoring of blood glucose)). The reference electrode can be prepared, as shown in the present embodiment, as a silver/silver chloride electrode provided with the silver/silver chloride (Ag/AgCl) layer 24. In addition, a mercury-containing layer can be formed, as in a hydrogen electrode, a calomel electrode, or the like.

The film 25 is a sheet-shaped insulating member formed (laminated) on a predetermined portion of the electrode 22 (the working electrode 22a, the reference electrode 22b, and the counter electrode 22c) formed on the substrate 21, and the silver/silver chloride layer 24. The thickness of the film 25 is usually 1 µm or larger and 150 µm or smaller, preferably 3 µm or larger and 50 µm or smaller, more preferably 5 µm or larger and 30 µm or smaller. The film 25 has openings in a portion corresponding to the reagent layer 23 located at the tip of the sensor 11, and a portion corresponding to a portion of the counter electrode 22c, and the reagent layer 23 and the counter electrode 22c in the portions are exposed.

The film 25 can be prepared, for example, by affixing a pressure-sensitive adhesive sheet (e.g., acrylic, rubber, or hot-melt type) to the same resin material as that of the substrate 21. The sheet of the resin material mentioned above may be a sheet of the same resin material as that of the substrate 21 described above or may be a sheet of a resin material different from that of the substrate 21. A single pressure-sensitive adhesive sheet may be used as the film 25. A thermoplastic or photoplastic resist film or a layer formed from resist ink may also be used as the film 25.

A reagent solution for forming the reagent layer 23 can be added dropwise and applied to the surface of the electrode 22 (the working electrode 22a) from the opening located at the corresponding portion of the film 25. The contact angle (α) of the reagent solution with respect to the surface of the film 25 is preferably higher than the contact angle (β) of the reagent solution with respect to the opening of the film 25, i.e., the exposed surface of the working electrode 22a, and the difference therebetween (α - β) is preferably larger, from the viewpoint of the workability of such a process, etc. For example, α is preferably 90° or larger, and β is preferably 50° or smaller. In this context, the "contact angle" is a "static contact angle" measured by the "θ/2 method". The material itself forming the film 25 and/or the material itself forming the exposed working electrode 22a may not satisfy the conditions of the contact angle as described above. Even in such a case, the conditions of the contact angle as described above may be satisfied by appropriate surface treatment, for example, at least one of water-repellent treatment of the film 25 and hydrophilic treatment of the working electrode 22a.

Although not shown in Figures 1(A) and 1(B), a portion comprising at least the reagent layer 23 of the sensor 11 may be coated with a protective film. Specifically, a protective film may be formed (laminated) on the upper face of the reagent layer 23 in Figure 2(B). The reagent layer will be mentioned later with reference to Figures 4 to 6 which show the reagent layer.

A method for producing the sensor 11 is not particularly limited, and a method that can produce the sensor 11 having the configurations as mentioned above at predetermined sites can be appropriately selected and used. The sensor 11 can be produced, for example, by performing the following steps (i) to (viii):
(i) forming (laminating) an electrode 22 on the upper face of a substrate 21;
(ii) forming grooves A1 and A2 which physically and electrically separate the electrode 22 into three regions a working electrode 22a, a reference electrode 22b, and a counter electrode 22c;
(iii) forming a silver/silver chloride layer 24 on the upper face of the electrode 22 (the reference electrode 22b) ;
(iv) forming (laminating) a film 25 on the upper faces of the electrode 22 and the silver/silver chloride layer 24;
(v) forming a reagent layer 23 on the upper face of the electrode 22 (the working electrode 22a);
(vi) removing a portion (region X6) of the reagent layer 23 and the electrode 22;
(vii) cutting the sensor 11 out of the substrate 21; and
(viii) forming a protective film.

In the step (i), a method for forming (laminating) the electrode 22 can be appropriately selected and adjusted in consideration of the combination of the material of the electrode 22 and the material of the substrate 21, etc. For example, when the material of the substrate 21 is a synthetic resin such as PET and the material of the electrode 22 is a metal, the electrode 22 made of the metal material can be formed on the surface of the substrate 21 by vapor deposition (including sputtering). Alternatively, printing, plating, spin coating, or the like may be used. When the material of the substrate 21 is a synthetic resin such as PET and the material of the electrode 22 is carbon, the electrode 22 made of carbon can be formed, for example, by printing a carbon paste on the surface of the substrate 21. The substrate 21 in this step does not have to have the shape of the sensor 11 in advance, and the substrate 21 having a larger dimension than that of the sensor 11 can be used such that the sensor 11 can be cut out by the step (vii) performed later.

In the step (ii), for example, laser trimming can be used as an approach of forming the grooves A1 and A2.

In the step (iii), a method for forming the silver/silver chloride layer 24 can be appropriately selected and adjusted in consideration of the combination of the material of the silver/silver chloride layer 24 and the material of the electrode 22, etc. The silver/silver chloride layer 24 can be formed on the upper face of the electrode 22, for example, by printing or applying a silver/silver chloride paste (ink) to the upper face of the electrode 22 made of a metal or carbon material by a method such as screen printing or ink jet, followed by drying. Alternatively, the silver/silver chloride layer 24 may be formed, for example, by printing, applying, or plating silver (Ag) to the upper face of the electrode 22, followed by chlorination treatment of the surface.

In the step (iv), a method for forming (laminating) the film 25 can be appropriately selected and adjusted in consideration of the combination of the material of the film 25 and the materials of the electrode 22 and the silver/silver chloride layer 24, etc. For example, an opening (having a larger dimension than that of at least the reagent layer 23) corresponding to the dimension of the reagent layer 23 is formed in the film 25 made of a laminate of a resin sheet and a pressure-sensitive adhesive sheet or made of a single pressure-sensitive adhesive sheet. The film 25 thus obtained is disposed on the upper face of the electrode 22 such that the opening surrounds a portion where the reagent layer 23 is to be formed in the electrode 22 (working electrode 22a). The film 25 is fixed thereon through the pressure-sensitive adhesive sheet. On the other hand, the film 25 having a predetermined opening can also be formed (laminated) by removing the portion of the predetermined position and dimension as mentioned above using a resist film or resist ink.

No film 25 is formed on the upper face of a portion (the region X3) of the counter electrode 22c in the electrode 22 to expose the counter electrode 22c. Thus, for example, the film may be temporarily formed, also including the upper face of the counter electrode 22c, as described above, and then, an opening in a cutout shape can be formed in the film 25 by cutting or the like.

In the step (v), for example, the film 25 having the predetermined opening and a reagent solution prepared in advance are used, and the reagent solution can be added dropwise to the opening portion of the film 25 formed (laminated) by the step (iv), and thereby applied to at least a portion of the working electrode 22a. Then, the applied reagent solution is dried to form the reagent layer 23 on the upper face of the working electrode 22a.

The opening of the film 25 may have, for example, a dimension and a shape that allow a reagent layer having a larger width than that of the sensor 11 (tip portion shown in Figure 1(B)) to be formed. In this case, a reagent layer formed with a larger width than that of the sensor 11 from the applied reagent solution is trimmed such that a predetermined width and shape are attained by the subsequent step (vi).

In the step (vi), the reagent layer 23 and the electrode 22 are removed (trimmed) over a predetermined length in the length direction (e.g., the direction of insertion to a living body) of the sensor 11 at an end in the width direction of the sensor 11 (tip portion). The reagent layer is formed over a certain area by such trimming in the step (vi). Then, a reagent layer having a predetermined area common among sensors is formed by selecting an appropriate portion (preferably a uniform portion). In addition, in the subsequent step (vii), the sensor 11 can be cut out of the substrate 21 along the contour without dividing the formed reagent layer. A method for removing the reagent layer 23 and the electrode 22 can be appropriately selected and adjusted in consideration of the material of the reagent layer 23 (the composition of the reagent solution) and the material of the electrode 22, etc. The reagent layer 23 and the electrode 22 can be removed by, for example, laser trimming.

In the step (vii), a method for cutting the sensor 11 out of the substrate 21 can be appropriately selected and adjusted in consideration of the material of the substrate 21, the shape of the sensor to be cut, etc. For example, when the material of the substrate 21 is a resin, a general cutting technique can be used.

The cutting position includes the portion trimmed by the step (vi) and can be set to, for example, near the center line of a bottom portion of a recess obtained by laser trimming. In other words, cutting is performed at a position distant to a certain degree from the trimmed reagent layer 23 and working electrode 22a.

The step (viii) can be performed with reference to the method for forming a protective film described in relation to the electrochemical sensor of the present invention, and in addition, can be appropriately selected and adjusted in consideration of the combination of the material of the protective film, i.e., the composition of a protective film solution, and the material of the portion to be coated (mainly, the material of the reagent layer 23, i.e., the composition of a reagent solution), the shape or area of the portion to be coated, etc. For example, the working electrode 22a (portion where at least the reagent layer 23 is formed) can be dipped in a protective film solution, pulled up, and dried to coat the portion with a protective film.

Figure 3 is a plane view illustrating a sensor 101 according to another embodiment of the present invention. The sensor 101 is constituted by a sensing portion (tip portion that is inserted to a living body or dipped in a medium) 121, and a terminal portion 122 for electric connection to the internal circuit of the main body (not shown).

Figure 4 shows a sectional view of the sensor 101 taken along the A-A' section line in Figure 3. Conductive thin films 112 are disposed on both sides of an insulating substrate 111. The conductive thin film 112 on one side (front side) of the insulating substrate 111 is separated into a working electrode region 112a and a reference electrode region 112b through electric isolation by forming a groove 113 having a depth that reaches the surface of the insulating substrate 111 by laser lithography.

The upper face of the insulating substrate 111 is coated with an insulating resist film 116a having an opening for forming a reference electrode 115 at a predetermined position of the reference electrode region 112b. The lower face of the insulating substrate 111 is coated with an insulating resist film 116b. On the other hand, neither the upper face nor the lower face is coated with the insulating resist film 116a or 116b from the end of the sensing portion 121 to a given distant location. A region 112a on the front side serves as a working electrode 114, and a region 112c on the back side serves as a counter electrode 117. A reagent layer 118 is formed on the working electrode 114.

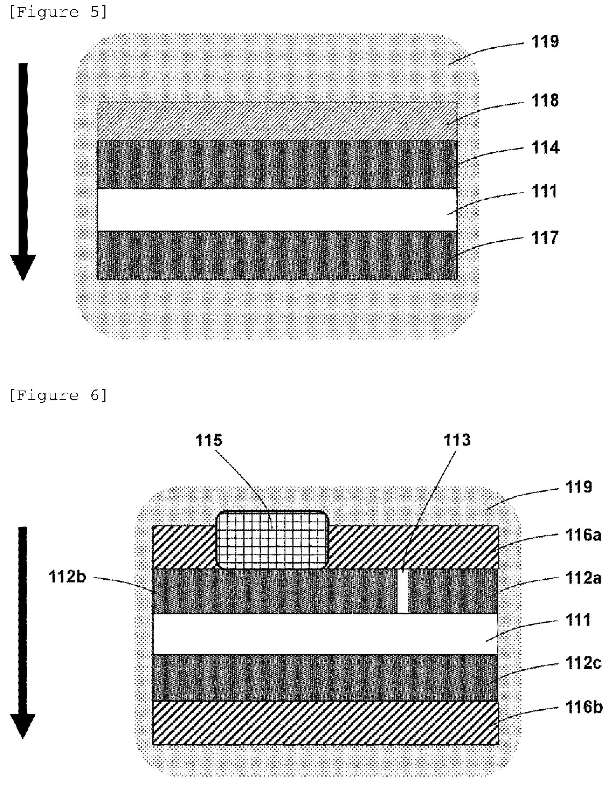

Figure 5 shows a sectional view taken along the B-B' section line in Figure 4. Figure 6 shows a sectional view taken along the C-C' section line in Figure 4. As shown in Figure 5, at the B-B' section line, a protective film 119, a reagent layer 118, a working electrode 114, an insulating substrate 111, a counter electrode 117, and a protective film 119 are formed in order from the upper face toward the lower face (in the direction of the arrow in the drawing). As shown in Figure 6, at the C-C' section line, a protective film 119, a reference electrode 115 (an insulating resist film 116a), a working electrode region 112a, an insulating substrate 111, a counter electrode region 112c, an insulating resist film 116b, and a protective film 119 are formed in order from the upper face toward the lower face (in the direction of the arrow in the drawing). The working electrode region 112a and the counter electrode region 112c shown in Figure 6 have insulating resist films 116a and 116b on the upper face side thereof and thus do not function as a working electrode or a counter electrode.

Figure 7 is a plane view illustrating a sensor 201 according to an alternative embodiment of the present invention. The sensor 201 comprises a sensing portion 202 having a configuration for electrochemically measuring an analyte, a terminal portion 203 having a configuration for electric connection to the internal circuit of the main body (not shown), and a cutout portion 204 provided in the vicinity of the terminal portion. The sensor 201 is an embodiment suitable for constructing a system for analyte measurement in a medium, for example. The sensing portion 202 has a structure dippable in a medium having a small liquid volume. The cutout portion 204 has a structure suitable for placing the sensor 201 in a culture container. Figure 7(A) shows an electrode pattern before formation of a film (insulating resist film) 230. Figure 7(B) shows an electrode pattern after formation of the film 230.

As shown in Figure 7(A), in the sensor 201, an electrode 220 is formed on a substrate 210. The electrode 220 is separated physically and electrically into a first working electrode 221, a second working electrode 222, a reference electrode 223, and a counter electrode 224 through a groove 225. As shown in Figure 7(B), the first working electrode 221 is coated with a film 230 and thereby has an exposed region 221a in the sensing portion 202 and an exposed region 221b in the terminal portion 203. Likewise, the second working electrode 222, the reference electrode 223, and the counter electrode 224 also respectively have exposed regions 222a, 223a, and 224a in the sensing portion 202 and exposed regions 222b, 223b, and 224b in the terminal portion 203. In the sensing portion 202, the exposed region 221a of the first working electrode and the exposed region 222a of the second working electrode are disposed side by side so as to maximize an area, while the exposed region 223a of the reference electrode and the exposed region 224a of the counter electrode are also disposed. Different types of reagent layers (not shown) for measuring different analytes can be formed on the exposed region 221a of the first working electrode and the exposed region 222a of the second working electrode, respectively. The exposed regions 221b, 222b, 223b, and 224b in the respective terminal portions 203 of the first working electrode, the second working electrode, the reference electrode, and the counter electrode can be electrically connected to the main body (not shown) through, for example, an electrode pad (not shown).

### Examples

The materials used in the present Examples (including Test Examples) are as follows.
(a) Conductive carbon filler
   (a-1) Carbon black ... Sigma-Aldrich Co., LLC, "05-1530"
(b) Anionic dispersant
   (b-1A) Poly(acrylic acid) 100,000 ... Sigma-Aldrich Co., LLC, "523925", average Mw: up to 100,000
   (b-1B) Poly(acrylic acid) 25,000 ... FUJIFILM Wako Pure Chemical Corp., "162-18581", average molecular weight: approximately 25,000
   (b-1C) Poly(acrylic acid) 5,000 ... FUJIFILM Wako Pure Chemical Corp., "165-18571", average molecular weight: approximately 5,000
   (b-2A) Poly(styrenesulfonic acid sodium salt) ... Sigma-Aldrich Co., LLC, "81609", average Mw: up to 16,800
   (b-2B) Poly(sodium 4-styrenesulfonate) ... Sigma-Aldrich Co., LLC, "243051", average Mw: up to 70,000
   (b-3) Poly(4-styrenesulfonic acid-co-maleic acid) sodium salt ... Sigma-Aldrich Co., LLC, "434558", average Mw: up to 20,000
   (b-4) Poly(ethylene oxide) -b-poly(acrylic acid) ... Polymer Source, Inc., "P6348-EOAA", Mn (POA-b-PAA): 2-b-2.4 (10³ g/mol)
   (b-5) Poly(styrene)-b-poly(acrylic acid) ... Polymer Source, Inc., "P2397-SAA", Mn (PS-b-PAA): 1.5-b-44 (10³ g/mol)
(Non-b) Non-anionic dispersant
   (Non-b-1) Hydroxypropylcellulose ... Nippon Soda Co., Ltd., "NISSO HPC"
(c) Cationic mediator
   (c-1) Polymer-bound PNT1 ... having a structure where a NHS form of a phenothiazine-based compound (PNT-70) as the redox mediator compound (c1) and a copolymer of 2-aminoethyl methacrylate hydrochloride, (4-vinylphenyl)methanamine, and methacryloylcholine chloride (hereinafter, referred to as "polymer C") as the cationic polymer (c2) were bound to each other.
   (c-2) Polymer-bound PNT2 ... having a structure where a phenothiazine-based compound (PNT-70) as the redox mediator compound (c1) and poly(L-lysine) hydrochloride (Alamanda Polymers Inc., "PLKC800") (hereinafter, referred to as "PLL") as the cationic polymer (c2) were bound to each other.

The PNT-70, the polymer C, the polymer-bound PNT1, and the polymer-bound PNT2 were respectively synthesized by the following methods.

### [Synthesis Example 1] Synthesis of PNT-70

### (1) Synthesis of sulfonic acid fragment

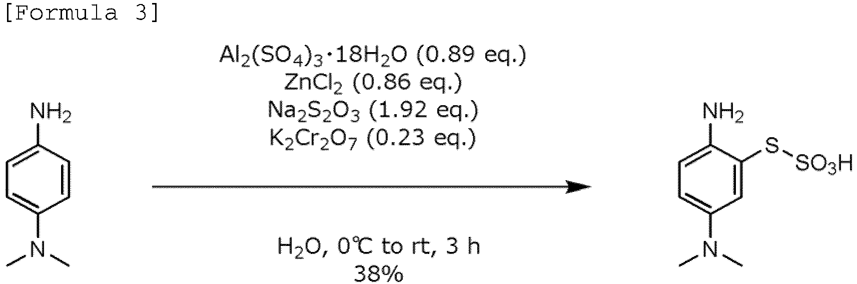

A sulfonic acid fragment was synthesized through the reaction described above.

### (2) Synthesis of sulfonic acid fragment

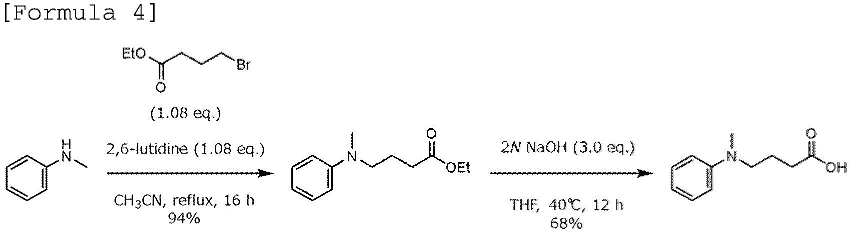

A carboxylic acid fragment was synthesized through two-stage reaction in acetonitrile and in tetrahydrofuran (THF) as described above.

### (3) Synthesis of PNT-34

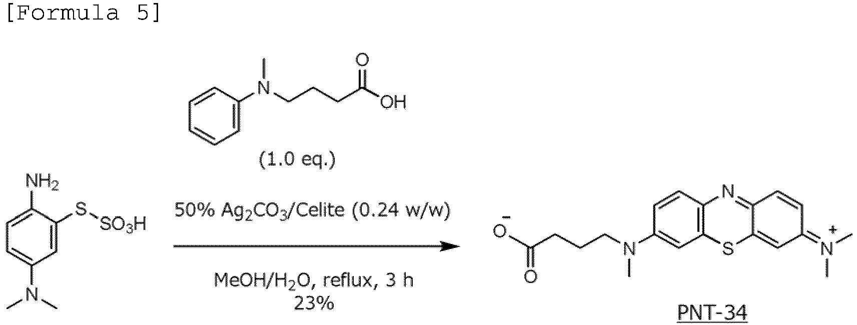

The sulfonic acid fragment and the carboxylic acid fragment synthesized as described above were suspended in MeOH/H₂O, and 50% Ag₂CO₃/celite was added thereto in divided portions over 15 minutes at an internal temperature around 47°C. After addition, the mixture was stirred at an internal temperature around 68°C for 2.5 hours. The reaction mixture was allowed to cool to room temperature and filtered through celite, and the filtrate was concentrated. The residue was purified a plurality of times by silica gel column chromatography to obtain PNT-34.

### (4) Synthesis of condensate (PNT-68)

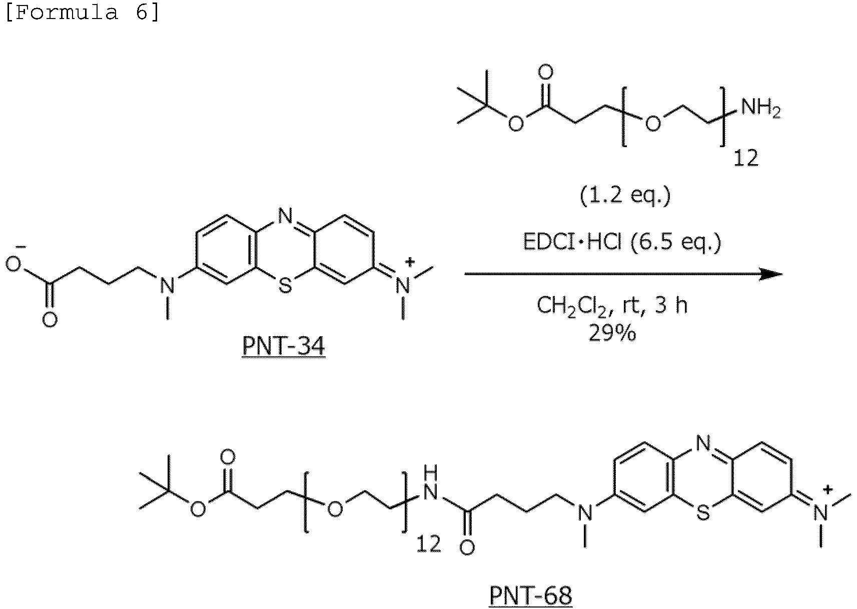

In an Ar atmosphere, PNT-34 was dissolved in dichloromethane. To the solution, amino-PEG12-t-butyl ester and 1-ethyl-3-(3-dimethylaminopropyl)carbodiimide hydrochloride (EDCI·HCl) were added, and the mixture was then stirred at room temperature for 3 hours. The reaction solution was concentrated and then purified by silica gel column chromatography three times to obtain a condensate (PNT-68).

### (5) Synthesis of deprotected form (PNT-69)

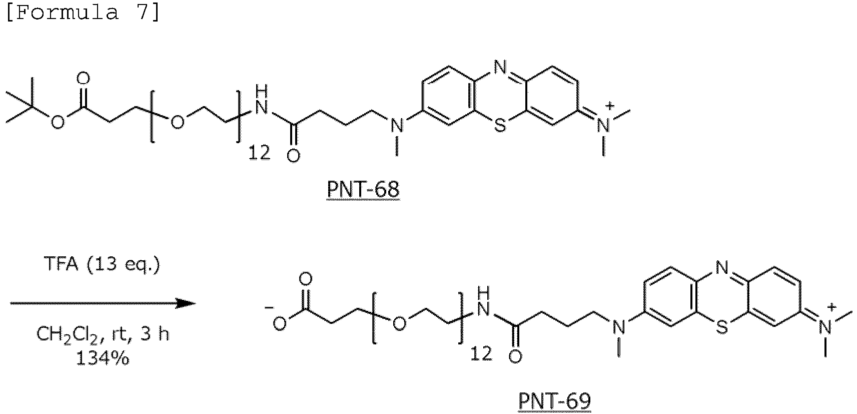

The condensate (PNT-68) obtained in (4) was dissolved in dichloromethane. To the solution, trifluoroacetic acid (TFA) was added, and the mixture was then stirred at room temperature for 3 hours. The reaction solution was concentrated and then subjected to azeotropy with toluene several times to obtain a deprotected form (PNT-69).

### (6) Synthesis of PNT-69NHS form (PNT-70)

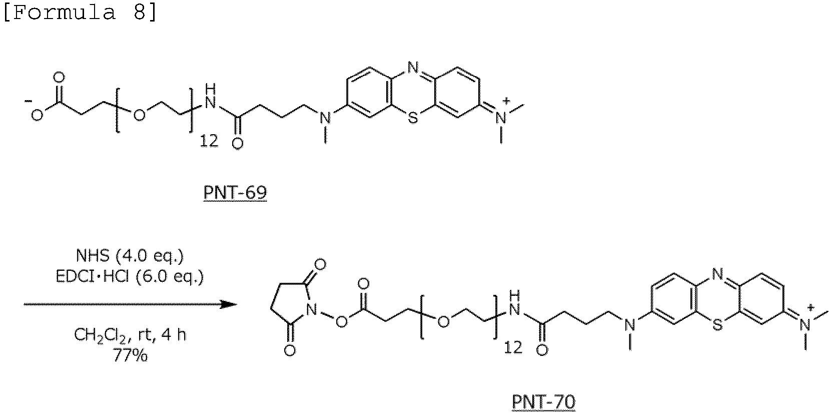

A deprotected form (PNT-69) was dissolved in dichloromethane. To the solution, N-hydroxysuccinimide (NHS) and EDCI·HCl were added, and the mixture was then stirred at room temperature for 4 hours. The reaction solution was purified by silica gel column chromatography to obtain a PNT-69NHS form having an activated (NHS esterified) carboxy group at the end of a PEG chain (PEG12) (PNT-70).

### [Synthesis Example 2] Synthesis of polymer C

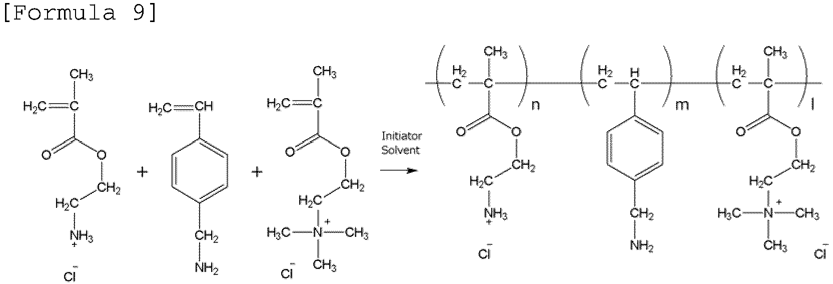

First, 2-aminoethyl methacrylate hydrochloride, (4-vinylphenyl)methanamine, and methacryloyl choline chloride were provided. Then, a four-neck flask was charged with 0.97 mmol of 2-aminoethyl methacrylate hydrochloride, 0.97 mmol of (4-vinylphenyl)methanamine, 11.97 mmol of methacryloyl choline chloride, 0.08 mmol of V-50, and 10.49 g of ethanol. Thereafter, the same treatment as in the polymer A was performed to obtain polymer C. The polymer C corresponds to a polymer with n:m:1 = 21.2:8.1:70.7 as a result of calculating a molar ratio from the results of 1H-NMR. The polymer had number-average molecular weight Mn of 37,774 and weight-average molecular weight Mw of 214,367 as a result of measurement by gel permeation chromatography (GPC).

### [Synthesis Example 3] Synthesis of polymer-bound PNT1

A PNT-69NHS form (PNT-70) was dissolved at 21.05 mg/mL in MilliQ water (liquid (1)). Polymer C was dissolved as a high-molecular-weight polymer at 15 mg/mL in MilliQ water (liquid (2)). Next, WSC (DOJINDO WO01) was dissolved at 20 mg/mL in MilliQ water (liquid (3)). 40 µL of the liquid (1), 140.3 µL of the liquid (2), 383.4 µL of the liquid (3), and 96 µL of a 250 mM 2-morpholinoethanesulfonic acid (MES) buffer solution (pH 6.0) prepared separately were mixed and adjusted to a total volume of 1200 µL with MilliQ water. Then, the mixture was reacted with stirring at room temperature for approximately 20 hours. Then, ultrafiltration was performed several times using a centrifugal ultrafiltration filter (Amicon Ultra-4 50k; Merck Millipore) to recover a liquid free from small molecules.

### [Synthesis Example 4] Synthesis of polymer-bound PNT2

A PNT-69NHS form (PNT-70) was dissolved at 15 mg/mL in MilliQ water (liquid (1)). Poly(L-lysine)hydrochloride (Alamanda Polymers Inc., PLKC800) was dissolved as a high-molecular-weight polymer at 10 mg/mL in MilliQ water (liquid (2)). Next, WSC (DOJINDO WO01) was dissolved at 20 mg/mL in MilliQ water (liquid (3)). 43.7 µL of the liquid (1), 51.3 µL of the liquid (2), 479.3 µL of the liquid (3), and 96 µL of a 250 mM 2-morpholinoethanesulfonic acid (MES) buffer solution (pH 6.0) prepared separately were mixed and adjusted to a total volume of 1200 µL with MilliQ water. Then, the mixture was reacted with stirring at room temperature for approximately 20 hours. Then, ultrafiltration was performed several times using a centrifugal ultrafiltration filter (Amicon Ultra-4 50k; Merck Millipore) to recover a liquid free from small molecules.

(d) Oxidoreductase
   (d-1) FAD-dependent glucose dehydrogenase ... GDH GLD1: manufactured by BBI International
(e) Cross-linking agent
   (e-1) Glutaraldehyde ... 25% glutaraldehyde solution (FUJIFILM Wako Pure Chemical Corp.)
(f) Buffer solution
   (f-1) Bis-Tris: (bis(2-hydroxyethyl)iminotris(hydroxymethyl)methane) ... manufactured by Dojindo Laboratories
   (f-2A) Tris-HCl pH 7.5 ... Invitrogen UltraPure 1 M Tris-HCl pH 7.5, Thermo Fisher Scientific, Inc.
   (f-2B) Tris-HCl pH 8.0 ... Invitrogen UltraPure 1 M Tris-HCl pH 8.0, Thermo Fisher Scientific, Inc.
   (f-2C) Tris-HCl pH 8.5 ... 1 M Tris-HCl (pH 8.5), FUJIFILM Wako Pure Chemical Corp. (manufacturer: Nippon Gene Co., Ltd.)
(g) Metal salt
   Sodium chloride (NaCl) ... FUJIFILM Wako Pure Chemical Corp. (product code: 191-01665)
   Potassium chloride (KCl) ... Nacalai Tesque, Inc. (product code: 28514-75)
   Sodium bromide (NaBr) ... FUJIFILM Wako Pure Chemical Corp. (product code: 192-09412)
   Potassium bromide (KBr) ... FUJIFILM Wako Pure Chemical Corp. (product code: 164-03472)
   Magnesium bromide (MgBr₂) heptahydrate ... FUJIFILM Wako Pure Chemical Corp. (product code: 138-09192, purity: 99.9%)
   Potassium sulfate (K₂SO₄) ... FUJIFILM Wako Pure Chemical Corp. (product code: 169-13552, purity: 99.9%)

### [Test Example 1] Dispersibility test

### <Preparation of reagent solution sample>

In accordance with the formulation shown in Table 2, the carbon black (a-1) was added at a carbon concentration of 20 mg/mL to aqueous solutions of various anionic dispersants (b) having a concentration of 1 mg/mL or 2 mg/mL, and the mixtures were then treated for 3 minutes or longer in an ultrasonic homogenizer to prepare reagent solution samples.

**[Table 2]**

| Reagent solution sample | (a) Conductive carbon filler | (b) Anionic dispersant | |
|---|---|---|---|
| 1-1 | (a-1) Carbon black | (b-1A) Poly(acrylic acid) 100,000 | 1 mg/mL |
| 1-2 | | (b-1B) Poly(acrylic acid)25,000 | 1 mg/mL |
| 1-3 | | (b-1C) Poly(acrylic acid)5,000 | 1 mg/mL |
| 1-4 | | (b-2A) Poly(styrenesulfonic acid sodium salt) | 1 mg/mL |
| 1-5 | | (b-2B) Poly(sodium 4-styrenesulfonate) | 1 mg/mL |
| 1-6 | | (b-3) Poly(4-styrenesulfonic acid-co-maleic acid) sodium salt | 1 mg/mL |
| | 20 mg/mL | | |
| 1-7 | | (b-4) Poly(ethylene oxide)-b-poly(acrylic acid) | 1 mg/mL |
| 1-8 | | (b-5) Poly(styrene)-b-poly(acrylic acid) | 1 mg/mL |
| 1-9 | | (b-1A) Poly(acrylic acid) 100,000 | 2 mg/mL |
| 1-10 | | (b-4) Poly(ethylene oxide)-b-poly(acrylic acid) | 2 mg/mL |
| 1-11 | | (b-5) Poly(styrene)-b-poly(acrylic acid) | 2 mg/mL |

### <Filter treatment of reagent solution>

The reagent solution samples obtained as described above were treated for approximately 10 minutes in an ultrasonic bath upon use. The obtained redispersion samples were filtered through a 0.8 µm filter (ADVANTEC, DISMIC 25CS080AN), and the recovered filtrates were diluted 10-fold with Milli-Q water to prepare recovered liquid samples.

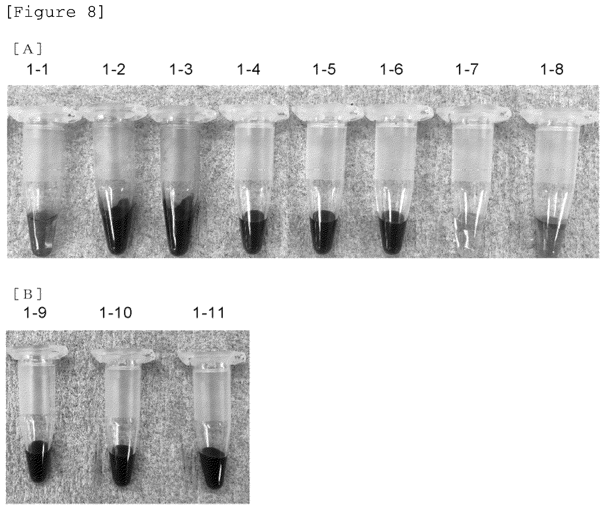

Figure 8 shows photographs of the recovered liquid samples. A lighter color of the recovered liquid samples means that less particles of the carbon black (a-1) passed through the filter and more particles of the aggregated carbon black (a-1) were captured by the filter, i.e., dispersibility was small. However, the anionic dispersants (b) in all the reagent solution (recovered liquid) samples 1-1 to 1-11 were found to have a dispersing effect on the conductive carbon filler (a) in an aqueous solvent in varying degrees. The results about the reagent solution (recovered liquid) samples 1-1 to 1-3 in which poly(acrylic acids) having different average molecular weights were used as the anionic dispersant (b) at the same concentration (1 mg/mL) suggest that in the case of using an anionic polymer such as poly(acrylic acid) as the anionic dispersant (b), the dispersing effect on the conductive carbon filler (a) is reduced with increase in its average molecular weight.

### [Test Example 2] pH and metal ion concentration dependence test - 1

In accordance with the formulation shown in Table 3, reagent solution samples containing predetermined concentrations of various components were prepared. The buffer solution used was prepared such that the pH of a reagent solution was a predetermined value when the buffer solution was used in a predetermined amount. In this Test Example 2, the pH was adjusted using a buffer solution containing no metal ion (metal salt), and dispersibility was then compared among the reagent solution samples, all of which contained the same concentration of a metal ion (metal salt).

The concentration of polymer-bound PNT was adjusted by first diluting a stock solution of each polymer-bound PNT 25-fold, adding the dilution at 100 µL to a microplate, measuring an absorption spectrum using a plate reader to confirm the concentration of the stock solution, and adjusting the concentration of each reagent sample on the basis of the obtained value. The value "corresponding to absorbance of 3" refers to approximately 0.12 at absorbance of 608 nm in the 25-fold dilution. Here, the microplate used was UV-Star(R) 96 Well F-Boden manufactured by Greiner Bio-One. The plate reader used was infinite(R) M200 Pro manufactured by Tecan Group Ltd.

**[Table 3]**

| Reagent solution sample | (a) Conductive carbon filler | (b) Anionic dispersant | (c) Cationic mediator | (f) Buffer solution | | | (g) Metal salt | Dispersibility evaluation 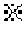 |
|---|---|---|---|---|---|---|---|---|
| 2-1 | | | | pH 6.0 | (f-1) Bis-Tris | 40 mM | Na 50 mM | ○ |
| 2-2 | (a-1) Carbon black | (b-1C) Poly(acrylic acid)5,000 | Polymer-bound PNT1 | pH 6.5 | (f-1) Bis-Tris | 40 mM | | ○ |
| 2-3 | | | | pH 7.0 | (f-1) Bis-Tris | 40 mM | | ○ |
| 2-4 | 3 mg/mL | 0.15 mg/mL | Corresponding to absorbance of 3 | pH 7.5 | (f-2A) Tris-HCl | 40 mM | (derived from 50 mM NaCl) | ○ |
| 2-5 | | | | pH 8.0 | (f-2B) Tris-HCl | 40 mM | | ○ |
| 2-6 | | | | pH 8.5 | (f-2C) Tris-HCl | 40 mM | | X |

| | | | | | | | | |
|---|---|---|---|---|---|---|---|---|
| 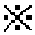 ○: Aggregation was absent ×: Aggregation occurred | | | | | | | | |

Each reagent solution sample was prepared as follows: the conductive carbon filler (a) was added to an aqueous solution of the anionic dispersant (b), and the mixture was treated for 3 minutes or longer in an ultrasonic homogenizer. The buffer solution (f) and the metal salt (g) were added to the resulting solution. Next, the cationic mediator (c) was added to the treated solution, and the mixture was treated for approximately 30 seconds in an ultrasonic homogenizer. The presence or absence of aggregation in the obtained reagent solution sample was visually confirmed. The results are also shown in Table 3.

### [Test Example 3] pH and metal ion concentration dependence test - 2

In accordance with the formulation shown in Table 4, reagent solution samples containing predetermined concentrations of various components were prepared. In this Test Example 3, the pH was adjusted using a buffer solution containing no metal ion (metal salt), and dispersibility was then compared among the reagent solution samples containing different concentrations of a metal ion (metal salt).

**[Table 4]**

| Reagent solution sample | (a) Conductive carbon filler | (b) Anionic dispersant | (c) Cationic mediator | (f) Buffer solution | (g) Metal salt | | Dispersibility evaluation 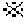 |
|---|---|---|---|---|---|---|---|
| 3-1 | | | | | Na (NaCl) | 0 mM | ○ |
| 3-2 | (a-1) Carbon black | (b-1C) Poly(acrylic acid)5, 000 | Polymer-bound PNT1 | pH 6.5 (f-1) | Na (NaGl) | 20 mM | ○ |
| 3-3 | | | | | Na (NaCl) | 50 mM | ○ |
| 3-4 | | | | Bis-Tris 40 mM | Na (NaCl) | 70 mM | ○ |
| 3-5 | 3 mg/mL | 0.15 mg/mL | Corresponding to absorbance of 3 | | Na (NaCl) | 80 mM | ○ |
| 3-6 | | | | | Na (NaCl) | 100 mM | X |

| | | | | | | | |
|---|---|---|---|---|---|---|---|
| 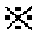 ○: Aggregation was absent ×: Aggregation occurred | | | | | | | |

Each reagent solution sample was prepared as follows: the conductive carbon filler (a) was added to an aqueous solution of the anionic dispersant (b), and the mixture was treated for 3 minutes or longer in an ultrasonic homogenizer. The buffer solution (f) and the metal salt (g) were added to the resulting solution. Next, the cationic mediator (c) was added to the treated solution, and the mixture was treated for approximately 30 seconds in an ultrasonic homogenizer. The presence or absence of aggregation in the obtained reagent solution sample was visually confirmed. The results are also shown in Table 4.

### [Test Example 4] pH and metal ion concentration dependence test - 3

In accordance with the formulation shown in Table 5, reagent solution samples containing predetermined concentrations of various components were prepared. In this Test Example 4, the pH was adjusted using a buffer solution containing no metal ion (metal salt), and dispersibility was then compared among the reagent solution samples containing different types and concentrations of a metal salt(metal ion).

**[Table 5]**

| Reagent solution sample | (a) Conductive carbon filler | (b) Anionic dispersant | (c) Cationic mediator | (f) Buffer solution | (g) Metal salt | | Dispersibility evaluation 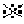 |
|---|---|---|---|---|---|---|---|
| 4-1 | | | | pH 6.5 | K (K₂SO₄) | 40 mM (20 mM) | ○ |
| 4-2 | | | | | Na (NaCl) | 50 mM | ○ |
| 4-3 | | | | | K (KCl) | 50 mM | ○ |
| 4-4 | | | | | Na (NaBr) | 50 mM | ○ |
| 4-5 | (a-1) Carbon black | (b - 1C) Poly(acrylic acid) 5,000 | Polymer-bound PNT1 | | K (KBr) | 50 mM | ○ |
| 4-6 | | | | | Mg (MgBr₂) | 50 mM | ○ |
| 4-7 | | | | (f-1) Bis-Tris | K (K₂SO₄) | 100 mM (50 mM) | X |
| 4-8 | 3 mg/mL | 0.15 mg/mL | Corresponding to absorbance of 3 | 40 mM | Na (NaCl) | 100 mM | X |
| 4-9 | | | | | K (KCl) | 100 mM | X |
| 4-10 | | | | | Na (NaBr) | 100 mM | X |
| 4-11 | | | | | K (KBr) | 100 mM | X |
| 4-12 | | | | | Mg (MgBr₂) | 100 mM | ○ |
| 4-13 | | | | | Mg (MgBr₂) | 200 mM | ○ |

| | | | | | | | |
|---|---|---|---|---|---|---|---|
| 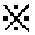 O:Aggregation was absent ×: Aggregation occurred | | | | | | | |

Each reagent solution sample was prepared as follows: the conductive carbon filler (a) was added to an aqueous solution of the anionic dispersant (b), and the mixture was treated for 3 minutes or longer in an ultrasonic homogenizer. The buffer solution (f) and the metal salt (g) were added to the resulting solution. Next, the cationic mediator (c) was added to the treated solution, and the mixture was treated for approximately 30 seconds in an ultrasonic homogenizer.

The presence or absence of aggregation in the obtained reagent solution sample was visually confirmed. The results are also shown in Table 5. As for the results of Test Examples 2 to 4, first, the results of Test Example 2 demonstrated that although the high pH of the reagent solution may cause aggregation (due to the influence of the pH itself, not the influence of the concentration of the metal salt derived from the buffer solution for pH adjustment), the pH range appropriate for the composition of the reagent solution can improve the dispersibility of the conductive carbon filler and suppress aggregation. The results of Test Examples 3 and 4 suggested that: although the occurrence status of aggregation differs depending on the type of the metal ion contained in the reagent solution, in general, aggregation tends to occur easily at a high concentration of the metal ion, and that a metal ion concentration of, for example, less than 100 mM can suppress aggregation for many metal ions, and on the other hand, demonstrated that even a concentration of higher than 50 mM (e.g., on the order of 200 mM), as in a magnesium ion, may suppress aggregation, and hence that the concentration range appropriate for the type of the metal ion can improve the dispersibility of the conductive carbon filler and suppress aggregation.

### [Test Example 5] Test on anionic dispersant (b)

In accordance with the formulation shown in Table 6, reagent solution samples containing predetermined concentrations of various components were prepared.

**[Table 6]**

| Reagent solution sample | (a) Conductive carbon filler | (b) Anionic dispersant | (c) Cationic mediator | | (d) Oxidore ductase | (e) Cross-linking agent | (f) Buffer solution | | | Dispersibility evaluation 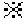 |
|---|---|---|---|---|---|---|---|---|---|---|
| 5-1 | | | (c-1) Polymer-bound PNT1 | Corresponding to absorbance of 3 | | | pH 5.3 | (f-1) Bis-Tris | 40 mM | ○ |
| 5-2 | | | | | | | pH 6.0 | (f-1) Bis-Tris | 40 mM | ○ |
| 5-3 | (a-1) Carbon black | (b-1C) Poly(acrylic acid) 5,000 | | Corresponding to absorbance of 4 | FAD-dependent glucose dehydrogenase | | pH 5.3 | (f-1) Bis-Tris | 40 mM | ○ |
| 5-4 | | | | | | Glutaral dehyde | pH 6.0 | (f-1) Bis-Tris | 40 mM | ○ |
| 5-5 | 3 mg/mL | 0.15 mg/mL | (c-2) Polymer-bound PNT2 | Corresponding to absorbance of 3 | 4000 U/mL | 0.01% (wt/v) | pH 5.3 | (f-1) Bis-Tris | 40 mM | X |
| 5-6 | | | | | | | pH 6.0 | (f-1) Bis-Tris | 40 mM | X |
| 5-7 | | | | Corresponding to absorbance of 4 | | | pH 5.3 | (f-1) Bis-Tris | 40 mM | ○ |
| 5-8 | | | | | | | pH 6.0 | (f-1) Bis-Tris | 40 mM | X |

| | | | | | | | | | | |
|---|---|---|---|---|---|---|---|---|---|---|
| 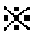 ○: Aggregation was absent ×: Aggregation occurred | | | | | | | | | | |

Each testing solution sample was prepared as follows: the conductive carbon filler (a) was added to an aqueous solution of the anionic dispersant (b), and the mixture was treated for 3 minutes or longer in an ultrasonic homogenizer. The buffer solution (f) was added to the resulting solution. Next, the cationic mediator (c) was added to the treated solution, and the mixture was treated for approximately 30 seconds in an ultrasonic homogenizer. Finally, the oxidoreductase (d) and the cross-linking agent (e) were added to the treated solution.

The presence or absence of aggregation in the obtained testing solution sample was visually confirmed. The results are also shown in Table 6. Polymer-bound PNT1 synthesized using a predetermined copolymer as the cationic polymer (c) and polymer-bound PNT2 synthesized using PLL as the cationic polymer (c) differed in an improving effect on the dispersibility of the conductive carbon filler (a) depending on the concentration in the reagent solution or the pH of the reagent solution. However, both the cationic polymers (c) were found to have the improving effect on the dispersibility of the conductive carbon filler (a) under some conditions.

### [Test Example 6] Response evaluation test

### <Preparation of reagent solution>

In accordance with the formulation shown in Table 7, reagent solution samples containing predetermined concentrations of various components were prepared.

**[Table 7]**

| Reagent solution sample | (a) Conductive carbon filler | (b) Anionic dispersant or control material thereof | | (c) Cationic mediator | (d) Oxidoreductase | (e) Cross-linking agent | (f) Buffer solution | Dispersibility evaluation 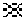 |
|---|---|---|---|---|---|---|---|---|
| 6-1 | | (b-1C) Poly(acrylic acid)5,000 | 0.15 mg/mL | | nase | | | ○ |
| 6-2 | | (b - 2A) Poly (styrenesulfonic acid sodium salt) | 0.15 mg/mL | | | | | ○ |
| 6-3 | | (b-3) Poly(4-styrenesulfonic acid-co-maleic acid) sodium salt | 0.15 mg/mL | | FAD-dependent glucose dehydroge | | pH 6.0 | ○ |
| | {a-1) Carbon black | | | Polymer-bound PNT1 | | Glutaraldehyde | Sodium phosphate buffer solution | |
| 6-4 | 3 mg/mL | (b-4) Poly(ethylene oxide)-b-poly(acrylic acid) | 0.15 mg/mL | Corresponding to absorbance of 7 | 4000 U/mL | 0.01% (wt/v) | | ○ |
| | | | | | | | 20 mM | |
| 6-5 | | (b-5) Poly(styrene)-b-poly(acrylic acid) | 0.15 mg/mL | | | | | ○ |
| 6-6 | | (Non-b) Hydroxypropylcellulose | 0.43 mg/mL | | | | | ○ |

| | | | | | | | | |
|---|---|---|---|---|---|---|---|---|
| 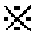 ○: Aggregation was absent ×: Aggregation occurred | | | | | | | | |

Each testing solution sample was prepared as follows: the conductive carbon filler (a) was added to an aqueous solution of the anionic dispersant (b) or the control material thereof, and the mixture was treated for 3 minutes or longer in an ultrasonic homogenizer. The buffer solution (f) was added to the resulting solution. Next, the cationic mediator (c) was added to the treated solution, and the mixture was treated for approximately 30 seconds in an ultrasonic homogenizer. Finally, the oxidoreductase (d) and the cross-linking agent (e) were added to the treated solution.

### <Preparation of electrode for sensor>

Each reagent solution sample prepared as described above was applied at 0.6 µL onto a carbon electrode prepared by screen printing on an insulating substrate, and dried overnight to obtain an electrode for a sensor.

### <Preparation of RPMI medium>

2-Morpholinoethanesulfonic acid (MES, manufactured by Dojindo Laboratories) or 3-morpholinopropanesulfonic acid (MOPS, manufactured by Dojindo Laboratories) were added as a buffer solution component to a solution prepared from RPMI-1640 Medium (R1383 manufactured by Sigma-Aldrich Co., LLC) such that the final concentration of each buffer solution component was 25 mM and the pH was adjusted to 7.4. The medium thus obtained was used as "RPMI medium" in Examples given below.

### <Sensor evaluation on response>

The electrode for a sensor as described above as a working electrode, a platinum electrode as a counter electrode, and a Ag/AgCl electrode (saturated KCl) (manufactured by BAS Inc.) as a reference electrode were combined to prepare a three-electrode measurement system. Time-dependent change in current (current response value) was measured in RPMI medium at 37°C by amperometry using a potentiostat (manufactured by BAS Inc.). Specifically, glucose was added at a theoretical value of 3 mM, 15 mM, or 30 mM every 100 seconds from 100 seconds after the start of measurement, and current response values were measured. The current response value was an average value calculated from five values measured on 5 seconds, 10 seconds, 15 seconds, 20 seconds, and 25 seconds immediately before the subsequent addition of glucose from after addition of glucose to be measured. The current value at the final concentration was an average value calculated from five values measured on 80 seconds, 85 seconds, 90 seconds, 95 seconds, and 100 seconds immediately before the subsequent addition of glucose from after addition of a glucose solution at a glucose concentration of 30 mM. The current value at each glucose concentration was a current value subjected to background correction treatment in which the current value at a glucose concentration of 0 mM was subtracted.

Cyclic voltammetry was performed in RPMI medium for the same three-electrode measurement system as above. A scan rate was set to 10 mV/s. Both the results of current response values and cyclic voltammetry were average measurement values from two sensors.

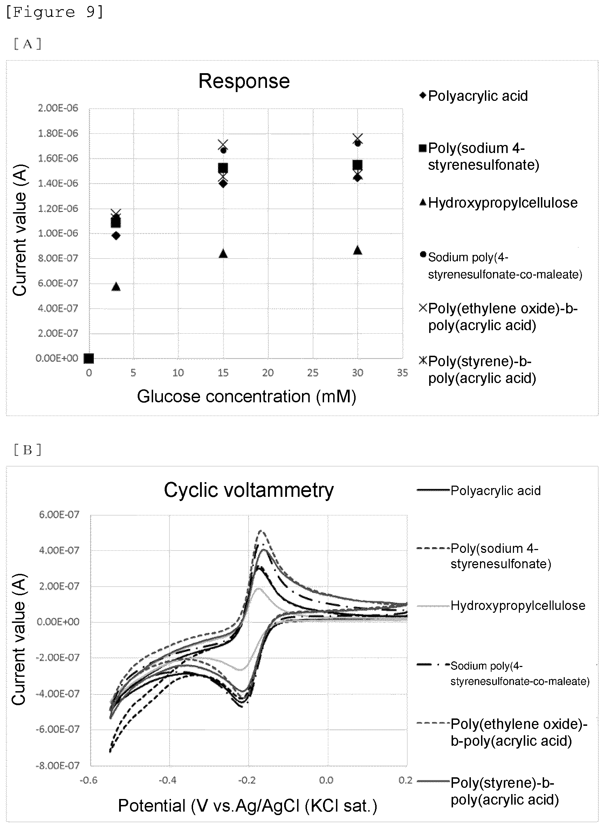

The results of current response values are shown in Figure 9[A], and the results of cyclic voltammetry are shown in Figure 9[B]. The current response exhibited a higher response in the sensors (reagent solution samples 6-1 to 6-5) prepared using each anionic dispersant (anionic polymer) than in the sensor (reagent solution sample 6-6) prepared using hydroxypropylcellulose. Likewise, the results of cyclic voltammetry also produced a higher redox peak value in the sensors prepared using each anionic dispersant (anionic polymer) than in the sensor prepared using hydroxypropylcellulose. This is presumably because use of the anionic dispersant (anionic polymer) enables the mediator to be closely adsorbed onto an electrode.

### [Test Example 7] Durability (response maintenance rate) evaluation test

### <Preparation of reagent solution>

In Test Example 7, the reagent solution samples 6-1 and 6-6 of Test Example 6 were prepared and used again.

### <Preparation of polymer solution for protective film>

Reagents shown below were mixed at the following final concentrations to prepare a polymer solution for a protective film.
Poly(ter.butyl methacrylate-b-4-vinylpyridine) (manufactured by Polymer Source, Inc.; hereinafter, referred to as "tBuMA4VP"), final concentration: 7.11% (wt/v)
Tripropylene glycol methyl ether methacrylate-styrene-4-vinylpyridine random copolymer (manufactured by NARD Institute, Ltd.; hereinafter, referred to as "TGMAS4VP"), final concentration: 0.89% (wt/v)
Poly(ethylene glycol) diglycidyl ether (manufactured by Sigma-Aldrich Co., LLC; hereinafter, referred to as "PEGDGE"), final concentration: 0.98% (wt/v)
HEPES buffer solution (pH 8.0), final concentration: 5 mM

tBuMA4VP, TGMAS4VP, and PEGDGE were dissolved in ethanol for use. The number-average molecular weight Mn of tBuMA4VP was 87,000 for poly(ter.butyl methacrylate) and 74,000 for poly(4-vinylpyridine). Mw/Mn of tBuMA4VP was 1.16. TGMAS4VP had tripropylene glycol methyl ether methacrylate:styrene:4-vinylpyridine = 6.6:20.3:73.0, number-average molecular weight Mn of 60,704, weight-average molecular weight Mw of 120,095, and Mw/Mn of 1.98. The number-average molecular weight Mn of PEGDGE was up to 1,000. The HEPES buffer solution was prepared using 2-[4-(2-hydroxyethyl)-1-piperazinyl]ethanesulfonic acid (manufactured by Dojindo Laboratories).

### <Preparation of electrode for sensor>

Each reagent solution sample prepared as described above was applied at 0.5 µL onto a gold electrode formed on an insulating substrate by sputtering, and dried for 15 minutes. This operation was repeated twice to perform application a total of three times. Then, the solution was dried overnight. A reagent layer corresponding to each reagent solution sample was thereby formed on each gold electrode. Each gold electrode provided with the reagent layer was dipped in the polymer solution for a protective film prepared as described above, pulled up, and dried. This operation was repeated a plurality of times to form a protective film on each gold electrode. This process produced an electrode for a sensor.

### <Sensor evaluation on durability (response maintenance rate) >

The electrode for a sensor prepared as described above as a working electrode, a platinum electrode as a counter electrode, and a Ag/AgCl electrode (saturated KCl) (manufactured by BAS Inc.) as a reference electrode were combined to prepare a three-electrode measurement system. Time-dependent change in current was measured in RPMI medium at 37°C by amperometry using a potentiostat (manufactured by BAS Inc.). Specifically, glucose was added at a theoretical value of 5 mM, 15 mM, or 30 mM every 1000 seconds from 1500 seconds after the start of measurement, and current response values were continuously measured. The electrode after measurement was preserved in RPMI medium of 37°C. The same measurement as above was performed on days 1, 2, and 3 after the start of preservation. The current value at the glucose concentration of 5 mM and 15 mM was an average value calculated from five values measured on 5 seconds, 10 seconds, 15 seconds, 20 seconds, and 25 seconds immediately before the subsequent addition of glucose from after addition of glucose to be measured. The current value at the final concentration was an average value calculated from five values measured on 980 seconds, 985 seconds, 990 seconds, 995 seconds, and 100 seconds after addition of a glucose solution at a glucose concentration of 30 mM. The current value at each glucose concentration was a current value subjected to background correction treatment in which the current value at a glucose concentration of 0 mM was subtracted.

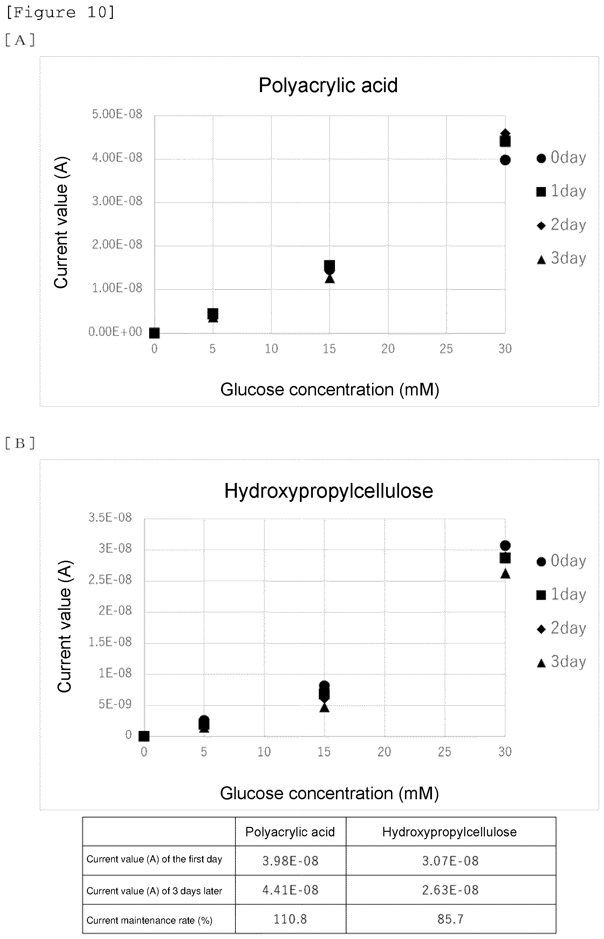

The results are shown in Figure 10. Even after coating with the protective film, the current response exhibited a higher value in the sensor prepared using poly(acrylic acid) (reagent solution sample 6-1) than in the sensor prepared using hydroxypropylcellulose (reagent solution sample 6-6). The sensor durability of 3 days later (current maintenance rate based on that of the first day) exhibited a higher value in the sensor prepared using poly(acrylic acid) (reagent solution sample 6-1). This is presumably because the mediator was more strongly adsorbed onto an electrode.

## Claims

1. A reagent layer comprising: a conductive carbon filler (a), an anionic dispersant (b), and a cationic mediator (c).

2. The reagent layer according to claim 1, wherein the anionic dispersant (b) is a polymer having a weight-average molecular weight of 70000 or smaller.

3. The reagent layer according to claim 1, wherein the anionic dispersant (b) is a polymer having a carboxy group and/or a sulfo group at a side chain.

4. The reagent layer according to claim 3, wherein the anionic dispersant (b) is a polymer comprising at least one member selected from the group consisting of an acrylic acid-derived unit, a maleic acid-derived unit, and a styrenesulfonic acid-derived unit.

5. The reagent layer according to claim 1, wherein the cationic mediator (c) is a compound in which a redox mediator compound (c1) and a cationic polymer (c2) are bound to each other optionally via a linker moiety (c3).

6. The reagent layer according to claim 5, wherein the cationic polymer (c2) has a quaternary ammonium cation group.

7. The reagent layer according to claim 1, wherein the conductive carbon filler (a) is carbon black.

8. The reagent layer according to claim 1, further comprising an oxidoreductase (e) that oxidizes or reduces an analyte.

9. The reagent layer according to claim 8, wherein the oxidoreductase (e) is bound to a coenzyme.

10. The reagent layer according to claim 8, wherein the oxidoreductase (e) is cross-linked to the cationic polymer (c2).

11. An electrochemical sensor for detecting or quantifying an analyte, the electrochemical sensor having a working electrode, a counter electrode, and the reagent layer according to any one of claims 1 to 10.

12. The electrochemical sensor according to claim 11, further having a reference electrode.

13. The electrochemical sensor according to claim 11, further having a protective film with which at least the reagent layer is coated.

14. A method for forming a reagent layer, comprising the steps of:
(1) preparing a reagent solution containing a conductive carbon filler (a), an anionic dispersant (b), and a cationic mediator (c);
(2) applying the reagent solution to a reagent layer formation site; and
(3) drying the applied reagent solution to form a reagent layer.

15. The method for forming a reagent layer according to claim 14, wherein a pH of the reagent solution is 8.0 or lower.

16. The method for forming a reagent layer according to claim 15, wherein a concentration of a metal ion in the reagent solution is 200 mM or less.

17. The method for forming a reagent layer according to claim 16, wherein the metal ion in the reagent solution is an alkali metal ion, and a concentration thereof is less than 100 mM.

18. The method for forming a reagent layer according to claim 14, wherein the anionic dispersant (b) is a polymer having a weight-average molecular weight of 70000 or smaller and comprising at least one member selected from the group consisting of an acrylic acid-derived unit, a maleic acid-derived unit, and a styrenesulfonic acid-derived unit.

19. The method for forming a reagent layer according to claim 14, wherein the cationic mediator (c) is a compound in which a redox mediator compound (c1) and a cationic polymer (c2) having a quaternary ammonium cation group are bound to each other optionally via a linker moiety (c3).
